# EUROPEAN PATENT SPECIFICATION

(11) **EP 1 127 057 B1**
(45) Date of publication and mention of the grant of the patent: **04.05.2005**
(21) Application number: 99947781.3
(22) Date of filing: 11.10.1999
(51) Int. Cl.: C07D 401/06, A61K 31/4725

(54) **NAPHTHYRIDINE COMPOUNDS AND THIER AZAISOSTERIC ANALOGUES AS ANTIBACTERIALS**
NAPHTHYRIDINE UND IHRE AZAISOSTEREN ANALOGA ALS ANTIBAKTERIELLE MITTEL
COMPOSES DE NAPHTYRIDINE ET LEURS ANALOGUES AZA-ISOSTERES UTILISES COMME AGENTS ANTIBACTERIENS

(30) Priority: 14.10.1998 GB 9822450
(43) Date of publication of application: 29.08.2001
(73) Proprietor: SmithKline Beecham plc, Brentford, Middlesex TW8 9GS (GB)
(72) Inventor: HATTON, Ian Keith, SmithKline Beecham Pharm., Harlow, Essex CM19 5AW (GB); PEARSON, Neil David, SmithKline Beecham Pharm., Harlow, Essex CM19 5AW (GB)
(74) Representative: Valentine, Jill Barbara
(86) International application number: PCT/GB1999/003366
(87) International publication number: WO 2000/021948

(56) References cited:
- EP-A- 0 030 044
- EP-A- 0 238 868
- EP-A- 0 304 493
- EP-A- 0 541 486
- EP-A- 0 823 429
- WO-A-95/09853
- WO-A-96/15128
- WO-A-97/03069
- WO-A-99/37635
- GB-A- 1 496 371
- KAYIRERE M -G ET AL: "Synthesis and antibacterial activity of new 4-alkoxy, 4-aminoalkyl and 4-alkylthioquinoline derivatives" EUROPEAN JOURNAL OF MEDICINAL CHEMISTRY.CHIMICA THERAPEUTICA,FR,EDITIONS SCIENTIFIQUE ELSEVIER, PARIS, vol. 33, no. 1, page 55-63 XP004173056 ISSN: 0223-5234

## Description

This invention relates to novel medicaments, being novel antibacterial compounds and compositions.

WO9937635, US4587341, GB1496371 and Kayirere, Eur. J. Med. Chem. 33 (1998) 55-63 disclose certain quinioline derivatives having antibacterial activity.

WO9217475, WO9802438, WO9703069 and WO9639145 disclose certain bicyclic heteroaromatic compounds having cholinesterase inhibitor, protein tyrosine kinase inhibitor, cell proliferation inhibitor and human epidermal growth factor receptor type 2 inhibitor activity.

WO9615128, EP0823429, EP0304493, EP0238868 disclose organic compounds having protein tyrosine kinase inhibitor and antibacterial activity.

WO9509853, EP0541486 and EP0374095 disclose organic compounds having protein kinase C inhibitor and antibacterial activity.

WO9728167 discloses organic compounds having bacterial histidine protein kinase inhibitor and antibacterial activity.

EP0030044 discloses quinoline derivatives having cardiovascular activity.

This invention provides a compound of formula (I) or a pharmaceutically acceptable derivative thereof: 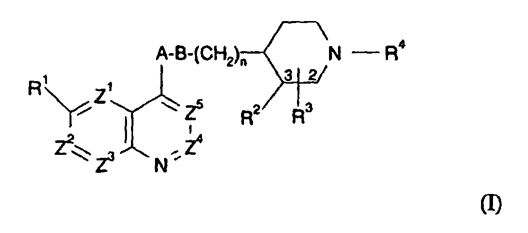 wherein:
one of Z¹, Z², Z³, Z⁴ and Z⁵ is N and the remainder are CH;
R¹ is hydrogen, hydroxy; (C₁₋₆) alkoxy optionally substituted by (C₁₋₆)alkoxy, amino, piperidyl, guanidino or amidino optionally N-substituted by one or two (C₁₋₆)alkyl, acyl or (C₁₋₆)alkylsulphonyl groups, NH₂CO, hydroxy, thiol, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted (C₁₋₆)alkyl; halogen; (C₁₋₆)alkyl; (C₁₋₆)alkylthio; nitro; trifluoromethyl; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups;
either R² is hydrogen; and
R³ is in the 2- or 3-position and is hydrogen or (C₁₋₆)alkyl or (C₂₋₆)alkenyl optionally substituted with 1 to 3 groups selected from:
   thiol; halogen; (C₁₋₆)alkylthio; trifluoromethyl; azido; (C₁₋₆)alkoxycarbonyl; (C₁₋₆)alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; amino optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl; aminocarbonyl wherein the amino group is optionally mono- or disubstituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl or (C₂₋₆)alkenylcarbonyl; oxo; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₂₋₆)alkenyl; or
R³ is in the 3-position and R² and R³ together are a divalent residue =CR^{5¹}R^{6¹} where R^{5¹} and R^{6¹} are independently selected from H, (C₁₋₆)alkyl, (C₂₋₆)alkenyl, aryl(C₁₋₆)alkyl and aryl(C₂₋₆)alkenyl, any alkyl or alkenyl moiety being optionally substituted by 1 to 3 groups selected from those listed above for substituents on R³;
R⁴ is a group -CH₂-R⁵ in which R⁵ is selected from:
   (C₃₋₁₂)alkyl; hydroxy(C₃₋₁₂)alkyl; (C₁₋₁₂)alkoxy(C₃₋₁₂)alkyl; (C₁₋₁₂)alkanoyloxy(C₃₋₁₂)alkyl; (C₃₋₆)cycloalkyl(C₃₋₁₂)alkyl; hydroxy-, (C₁₋₁₂)alkoxy- or (C₁₋₁₂)alkanoyloxy-(C₃₋₆)cycloalkyl(C₃₋₁₂)alkyl; cyano(C₃₋₁₂)alkyl; (C₂₋₁₂)alkenyl; (C₂₋₁₂)alkynyl; tetrahydrofuryl; mono- or di-(C₁₋₁₂)alkylamino(C₃₋₁₂)alkyl; acylamino(C₃₋₁₂)alkyl; (C₁₋₁₂)alkyl- or acyl-aminocarbonyl(C₃₋₁₂)alkyl; mono- or di- (C₁₋₁₂)alkylamino(hydroxy) (C₃₋₁₂)alkyl; optionally substituted phenyl(C₁₋₂)alkyl, phenoxy(C₁₋₂)alkyl or phenyl(hydroxy)(C₁₋₂)alkyl; optionally substituted diphenyl(C₁₋₂)alkyl; optionally substituted pheayl(C₂₋₃)alkenyl; optionally substituted benzoyl or benzoylmethyl; optionally substituted heteroaryl(C₁₋₂)alkyl;and optionally substituted heteroaroyl or heteroaroylmethyl:
n is 0, 1 or 2;
A is NR¹¹, O, S(O)ₓ or CR⁶R⁷ and B is NR¹¹, O, S(O)ₓ or CR⁸R⁹ where x is 0, 1 or 2 and wherein:
   each of R⁶ and R⁷, R⁸ and R⁹ is independently selected from: H; thiol; (C₁₋₆)alkylthio; halo; trifluoromethyl; azido; (C₁₋₆)alkyl; (C₂₋₆)alkenyl; (C₁₋₆)alkoxycarbonyl; (C₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³;
   (C₁₋₆)alkylsulphonyl; (C₂₋₆)alkenylsulphonyl; or (C₁₋₆)aminosulphonyl wherein the amino group is optionally substituted by (C₁₋₆)alkyl or (C₁₋₆)alkenyl;
   or R⁶ and R⁸ together represent a bond and R⁷ and R⁹ are as above defined;
   or R⁶ and R⁸ together represent -O- and R⁷ and R⁹ are both hydrogen;
   or R⁶ and R⁷ or R⁸ and R⁹ together represent oxo;
   and each R¹¹ is independently H, trifluoromethyl, (C₁₋₆)alkyl, (C₁₋₆)alkenyl, (C₁₋₆)alkoxycarbonyl, (C₁₋₆)alkylcarbonyl, aminocarbonyl wherein the amino group is optionally mono- or di-substituted by (C₁₋₆)alkoxycarbonyl, (C₁₋₆)alkylcarbonyl, (C₁₋₆)alkenyloxycarbonyl, (C₂₋₆)alkenylcarbonyl, (C₁₋₆)alkyl or (C₁₋₆)alkenyl;
provided that A and B cannot both be selected from NR¹¹, O and S(O)ₓ and when one of A and B is CO the other is not CO, O or S(O)ₓ;
wherein
'heterocyclic' is an optionally substituted aromatic or non-aromatic, single or fused, ring containing up to four hetero-atoms in each ring selected from oxygen, nitrogen and sulphur, and having from 4 to 7 ring atoms, which rings may be unsubstituted or substituted by up to three groups selected from amino, halogen, (C₁₋₆)alkyl, (C₁₋₆)alkoxy, halo(C₁₋₆)alkyl, hydroxy, carboxy, carboxy salts, (C₁₋₆)alkoxycarbonyl, (C₁₋₆)alkoxycarbonyl(C₁₋₆)alkyl, aryl, and oxo groups, and wherein any amino group forming part of a single or fused non-aromatic heterocyclic ring as defined above is optionally substituted by (C₁₋₆)alkyl optionally substituted by hydroxy, (C₁₋₆)alkoxy, thiol, (C₁₋₆)alkylthio, halo or trifluoromethyl, acyl and (C₁₋₆)alkylsulphonyl groups;
'aryl' is optionally substituted phenyl or naphthyl
optionally substituted phenyl or naphthyl is optionally substituted with up to five groups selected from halogen, mercapto, (C₁₋₆)alkyl, phenyl, (C₁₋₆)alkoxy, hydroxy(C₁₋₆)alkyl, mercapto (C₁₋₆)alkyl, halo(C₁₋₆)alkyl, hydroxy, amino, nitro, carboxy, (C₁₋₆)alkylcarbonyloxy, (C₁₋₆)alkoxycarbonyl, formyl and (C₁₋₆)alkylcarbonyl groups;
'acyl' is (C₁₋₆)alkoxycarbonyl, formyl or (C₁₋₆) alkylcarbonyl.

In one aspect the invention provides compounds of formula (I) where R 1 is selected from the groups listed above other than trifluoromethyl.

The invention also provides a method of treatment of bacterial infections in mammals, particularly in man, which method comprises the administration to a mammal in need of such treatment of an effective amount of a compound of formula (I) or a pharmaceutically acceptable derivative thereof.

The invention also provides the use of a compound of formula (I) or a pharmaceutically acceptable derivative thereof in the manufacture of a medicament for use in the treatment of bacterial infections in mammals.

The invention also provides a pharmaceutical composition for use in the treatment of bacterial infections in mammals comprising a compound of formula (I), or a pharmaceutically acceptable derivative thereof, and a pharmaceutically acceptable carrier.

Preferably Z¹ is N and Z²-Z⁵ are each CH or Z⁵ is N and Z¹-Z⁴ are each CH.

In a preferred aspect, when A is CH₂ or CHOH and B is CH₂ or A is CH₂ and B is CHOH and n is 1 the substitutents at the 3- and 4-position of the piperidine ring are cis.

When R¹ is substituted alkoxy it is preferably C₂₋₆ alkoxy substitituted by optionally N-substituted amino, guanidino or amidino, more preferably by amino, or C₁₋₆alkoxy substituted by piperidyl. Suitable examples of R¹ alkoxy include methoxy, n-propyloxy, i-butyloxy, aminoethyloxy, aminopropyloxy, aminopentyloxy, guanidinopropyloxy, piperidin-4-ylmethyloxy or 2-aminocarbonylprop-2-oxy. Preferably R¹ is methoxy, amino(C₃₋₅)alkyloxy, guanidino(C₃₋₅)alkyloxy or fluoro, most preferably methoxy.

R³ is preferably (C₁₋₆) alkyl, (C₁₋₆) alkenyl, aminocarbonyl(C₁₋₆) alkyl or optionally substituted 1-hydroxy-(C₁₋₆) alkyl, more preferably hydroxymethyl or 1,2-dihydroxy(C₂₋₆)alkyl wherein the 2-hydroxy group is optionally substituted. Preferred examples of R³ include aminocarbonylmethyl, hydroxymethyl, 1- hydroxyethylor 1,2-dihydroxyethyl wherein the 2-hydroxy group is optionally substituted with alkylcarbonyl or aminocarbonyl where the amino group is optionally substituted. Other suitable examples of R³ include 2-hydroxyethyl, 2- or 3-hydroxypropyl, ethyl or vinyl.

R3 is preferably in the 3-position.

When R² and R³ together form a group, this is preferably =CHCH₃.

Preferably A is NH, NCH₃, O, CH₂, CHOH, CH(NH₂), C(Me)(OH) or CH(Me).

Preferably B is CH₂, CHOH, CO or S.

Alternatively and preferably, A is CR⁶R⁷ and B CR⁸R⁹ and R⁶ and R⁸ together represent -O- and R⁷ and R⁹ are both hydrogen.

Preferably n is 0 or 1.

More preferably:
when A is NH, B is CO and n is 1 or 0;
when A is O, B is CH₂ and n is 1 or 0;
when A is CH₂ or CH₂OH, B is CH₂, and n is 1 or 0;
when A is NCH₃, CH(NH₂), C(Me)(OH) or CH(Me), B is CH₂ and n is 1;
when A is CR⁶R⁷ and B CR⁸R⁹ and R⁶ and R⁸ together represent -O- and R⁷ and R⁹ are both hydrogen and n is 1.

More preferably AB(CH₂)ₙ is CH₂CH₂, CH(OH)CH₂, CH₂CH(OH) or NHCO and n is 0 or 1. Most preferably AB is NHCO, NHCOCH₂ or CH₂CH(OH)CH₂.

Suitable groups R⁴ include n-pentyl, n-hexyl, n-heptyl, n-octyl, n-nonyl, n-decyl, n-dodecyl, methoxybutyl, phenylethyl, phenylpropyl or 3-phenyl-prop-2-en-yl optionally substituted on the phenyl ring, 3-benzoylpropyl, 4-benzoylbutyl, 3-pyridylmethyl, 3-(4-fluorobenzoyl)propyl, cyclohexylmethyl, cyclobutylmethyl, t-butoxycarbonylaminomethyl and phenoxyethyl.

Preferably R⁴ is (C₅₋₁₀)alkyl, unsubstituted phenyl(C₂₋₃)alkyl or unsubstituted phenyl(C₃₋₄)alkenyl, more preferably hexyl, heptyl, 5-methylhexyl, 6-methyl heptyl, 3-phenyl-prop-2-en-yl or 3-phenylpropyl, most preferably n-heptyl.

Most preferably R⁵ is unbranched at the α and, where appropriate, β positions.

Halo or halogen includes fluoro, chloro, bromo and iodo.

Each heterocyclic ring preferably has 5 or 6, ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

Examples of aromatic heterocyclic groups referred to above include pyridyl, triazolyl, tetrazolyl, indolyl, thienyl, isoimidazolyl, thiazolyl, furanyl,quinolinyl, imidazolidinyl and benzothienyl.

Phenyl and naphthyl may be optionally substituted with preferably up to three groups as defined.

Compounds of formula (I) wherein:
R³ is aminocarbonyl(C₁₋₆) alkyl, hydroxy(C₁₋₆)alkyl or 1,2-dihydroxy(C₂₋₆)alkyl optionally substituted on the hydroxy group(s) as claimed, hereinafter 'compounds of formula (IA)', are particularly preferred.

Compounds of formula (I) wherein:
R¹ is C₂₋₆ alkoxy substituted by optionally N-substituted amino, guanidino or amidino or C₁₋₆ alkoxy substituted by piperidyl, A is NH, CH₂, CHOH, CH(NH₂), C(Me)(OH) or CH(Me) and B is CH₂, CHOH or CO, hereinafter 'compounds of formula (IB)', form another aspect of the invention.

The invention also provides a pharmaceutical composition comprising a compound of formula (I), or a pharmaceutically acceptable derivative thereof, and a pharmaceutically acceptable carrier.

Some of the compounds of this invention may be crystallised or recrystallised from solvents such as organic solvents. In such cases solvates may be formed. This invention includes within its scope stoichiometric solvates including hydrates as well as compounds containing variable amounts of water that may be produced by processes such as lyophilisation.

Since the compounds of formula (I) are intended for use in pharmaceutical compositions it will readily be understood that they are each provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 59% of a compound of the formula (I) or salt thereof.

Pharmaceutically acceptable derivatives of the above-mentioned compounds of formula (1) include the free base form or their acid addition or quaternary ammonium salts, for example their salts with mineral acids e.g. hydrochloric, hydrobromic or sulphuric acids, or organic acids, e.g. acetic, fumaric or tartaric acids. Compounds of formula (I) may also be prepared as the N-oxide.

Certain of the above-mentioned compounds of formula (I) may exist in the form of optical isomers, e.g. diastereoisomers and mixtures of isomers in all ratios, e.g. racemic mixtures. The invention includes all such forms, in particular the pure isomeric forms. For examples the invention includes compound in which an A-B group CH(OH)-CH₂ is in either isomeric configuration.

In a further aspect of the invention there is provided a process for preparing compounds of formula (I), or a pharmaceutically acceptable derivative thereof, which process comprises:
(a) reacting a compound of formula (IV) with a compound of formula (V): 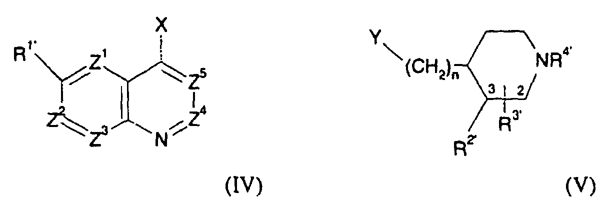 wherein Z¹, Z², Z³, Z⁴ and Z⁵, m, n, R¹, R², R³ and R⁴ are as defined in formula (I), and X and Y may be the following combinations:
   (i) X is M and Y is CH₂CO₂R^{x}
   (ii) X is CO₂R^{y} and Y is CH₂CO₂R^{x}
   (iii) one of X and Y is CH=SPh₂ and the other is CHO
   (iv) X is CH₃ and Y is CHO
   (v) X is CH₃ and Y is CO₂R^{x}
   (vi) X is CH₂CO₂R^{y} and Y is CO₂R^{x}
   (vii) X is CH=PR^{z}₃ and Y is CHO
   (viii) X is CHO and Y is CH=PR^{z}₃
   (ix) X is halogen and Y is CH=CH₂
   (x) one of X and Y is COW and the other is NHR^{11'}
   (xi) one of X and Y is (CH₂)ₚ-V and the other is (CH₂)_{q}NHR^{11'}, (CH₂)_{q}OH, (CH₂)_{q}SH or (CH₂)_{q}SCOR^{x} where p+q=1
   (xii) one of X and Y is CHO and the other is NHR^{11'}
   (xiii) one of X and Y is OH and the other is -CH=N₂
   in which V and W are leaving groups, R^{x} and R^{y} are (C₁₋₆)alkyl and R^{z} is aryl or (C₁₋₆)alkyl;
   or
(b) reacting a compound of formula (IV) with a compound of formula (Vb): 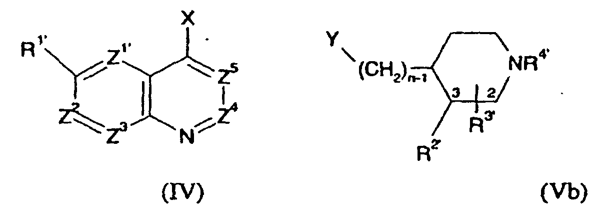 wherein Z¹, Z², 2³, Z⁴ and Z⁵, m, n, R¹, R², R³ and R⁴ are as defined in formula (I), X is CH₂NHR^{11'} and Y is CHO or COW or X is CH₂OH and Y is -CH=N₂;
   in which R^{11'}, R^{1'}, R^{2'}, R^{3'} and R^{4'} are R¹¹, R¹, R², R³ and R⁴ or groups convertible thereto, and thereafter optionally or as necessary converting R^{11'}, R^{1'}, R^{2'}, R^{3'} and R^{4'} to R¹¹, R¹, R², R³ and R⁴, converting A-B to other A-B, interconverting R¹¹, R¹, R², R³ and/or R⁴ and forming a pharmaceutically acceptable derivative thereof.

Process variants (a)(i) and (a)(ii) initially produce compounds of formula (I) where A-B is COCH₂.

Process variant (a)(iii) initially produces compounds of formula (I) wherein A-B is CH₂CHOH or CHOHCH₂.

Process variant (a)(iv) initially produces compounds of formula (I) wherein A-B is CH₂CHOH.

Process variants (a)(v) and (a)(vi), initially produce compounds of formula (I) wherein A-B is CH₂CO.

Process variants (a)(vii), (a)(viii) and (a)(ix) initially produce compounds where A-B is CH=CH.

Process variant (a)(x) initially produces compounds of formula (1) wherein A-B is CONHR¹¹ or NHR¹¹CO.

Process variant (a)(xi) initially produces compounds of formula (I) wherein one of A and B is CH₂ and the other is NHR¹¹, O or S.

Process variant (a)(xii), initially produce compounds of formula (I) wherein A-B is CH₂NHR¹¹ or NHR¹¹CH₂.

Process variant (a)(xiii) initially produces compounds of formul a(I) wherein A-B is OCH₂ or CH₂O.

Process variant (b) initially produces compounds of formula (I) wherein Al is CH₂ and B is NHR¹¹ or O.

In process variant (a)(i) M is preferably an alkali metal, more preferably Li. The reaction is conducted in an aprotic solvent preferably THF, ether or benzene at -78 to 25°C. An analogous route is described in G. Grethe et al (1972) Helv. Chimica.Acta., 55, 1044.

In process variant (a)(ii) the process is two step: firstly a condensation using a base, preferably sodium hydride or alkoxide, sodamide, alkyl lithium or lithium dialkylamide, preferably in an aprotic solvent e.g. ether, THF or benzene; secondly, hydrolysis using an inorganic acid, preferably HCl in aqueous organic solvent at 0-100°C. Analogous routes are described in DE330945, EP31753, EP53964 and H. Sargent, J. Am. Chem. Soc. **68,** 2688-2692 (1946).

In process variant (a)(iii) if a base is used it is preferably NaH, KH, an alkyl lithium e.g. BuLi, a metal alkoxide e.g. NaOEt, sodamide or lithium dialkylamide e.g.diisopropylamide. An analogous method is described in US 3989691 and in Taylor et al. (1972) JACS 94, 6218)

In process variant (a)(iv) the reaction is carried out in the presence of a base, preferably organometallic or metal hydride e.g. NaH, lithium diisopropylamide or NaOEt, preferably in an aprotic solvent, preferably THF, ether or benzene at -78 to 25°C (analogous process in Gutswiller et al. (1978) JACS 100, 576).

In process variant (a)(v) the reaction is carried out in the presence of a base, preferably organometallic or metal hydride e.g. NaH, lithium diisopropylamide or NaOEt, preferably in an aprotic solvent. preferably THF, ether or benzene at -78 to 25°C. An analogous method is described in US 3772302.

In process variant (a)(vi) a similar Claisen methodology to that described for (a)(ii) is used, analogous to that described in Soszko et. al., Pr.Kom.Mat. Przyr.Poznan.Tow.Przyj.Nauk., (1962), 10, 15.

In process variants (a)(vii) and (viii) if a base is used it is preferably NaH, KH, an alkyl lithium e.g. BuLi, a metal alkoxide e.g. NaOEt, sodamide or lithium dialkylamide e.g.di- isopropylamide. An analogous method is described in US 3989691 and M.Gates et. al. (1970) J. Amer.Chem.Soc., 92, 205, as well as Taylor et al. (1972) JACS 94, 6218.

In process variant (a)(ix) the reaction is carried out using palladium catalysis. The palladium catalyst is preferably palladium acetate in the presence of trialkyl or triaryl phosphine and a trialkylamine e.g. triphenyl phosphine and tributylamine. An analogous method is described in S. Adam et. al. (1994) Tetrahedron, 50, 3327.

In process variant (a)(x), or (b) where Y is COW,the reaction is a standard amide formation reaction:
1. Activation of a carboxylic acid (e.g., to an acid chloride, mixed anhydride, active ester, O-acyl-isourea or other species), and treatment with an amine (Ogliaruso, M. A.; Wolfe, J. F. in *The Chemistry of Functional Groups (Ed. Patai, S.) Suppl. B: The Chemistry of Acid Derivatives, Pt. 1* (John Wiley and Sons, 1979), pp 442-8; Beckwith, A. L. J. in *The Chemistry of Functional Groups (Ed. Patai, S.) Suppl. B: The Chemistry ofAmides (Ed. Zabricky, J*.) (John Wiley and Sons, 1970), p 73 ff. The acid and amide are preferably reacted in the presence of an activating agent such as 1-(dimethylaminopropyl)-3-ethylcarbodiimide hydrochloride (EDC) or 1-hydroxybenzotriazole (HOBT),
2. Aminolysis of esters (Suzuki, K.; Nagasawa, T. in *Encyclopedia of Reagents for Organic Synthesis (Ed. Paquette, L. A.*) (John Wiley and Sons, 1995), p 5188 and refs. cited therein.)
3. The specific methods of:
   a. *in situ* conversion of an acid into the amine component by a modified Curtius reaction procedure (Shioiri, T.; Murata. M.; Hamada, Y., *Chem. Pharm. Bull*. **1987,** *35*, 2698)
   b. *in situ* conversion of the acid component into the acid chloride under neutral conditions (Villeneuve, G. B.; Chan, T. H., *Tet. Lett.* **1997,** *38*, 6489).

In process variant (b) a final reduction step provides the required amine.

In process variant (a)(xi) where one of X and Y contains NHR¹¹ the leaving group V is halogen and the reaction is a standard amine formation reaction such as direct alkylation described in (Malpass. J. R., in *Comprehensive Organic Chemistry*, Vol. 2 (Ed. Sutherland, I. O.), p 4 ff.) or aromatic nucleophilic displacement reactions (see references cited in *Comprehensive Organic Chemistry*, Vol. 6, p 946-947 (reaction index); Smith, D. M. in *Comprehensive Organic Chemistry*, Vol. 4 (Ed. Sammes, P. G.) p 20 ff.). This is analogous to the methods described in GB 1177849.

In process variant (a)(xi) where one of X and Y contains OH or SH, this is preferably converted to an OM or SM group where M is an alkali metal by treatment of an alcohol, thiol or thioacetate with a base. The base is preferably inorganic such as NaH, lithium diisopropylamide or sodium, or, for SH, metal alkoxide such as sodium methoxide. The X/Y group containing the thioacetate SCOR^{x} is prepared by treatment of an alcohol or alkyl halide with thioacetic acid or a salt thereof under Mitsunobu conditions. The leaving group V is a halogen. The reaction may be carried out as described in Chapman et.al., J. Chem Soc., (1956),1563, Gilligan et. al., J. Med. Chem., (1992), **35**, 4344, Aloup et. al., J. Med. Chem. (1987), **30**, 24, Gilman et al., J.A.C.S. (1949), **71,** 3667 and Clinton et al., J.A.C.S. (1948), **70**, 491, Barluenga et al., J. Org. Chem. (1987) **52,** 5190. Alternatively where X is OR and Y is CH₂V, V is a hydroxy group activated under Mitsunobu conditions (Fletcher et.al. J Chem Soc. (1995), 623).

In process variants (a)(xii) and (b) where Y is CHO the reaction is a standard reductive alkylation using, e.g., sodium triacetoxyborohydride (Gribble, G. W. in *Encyclopedia of Reagents for Organic Synthesis (Ed. Paquette, L. A.)* (John Wiley and Sons, 1995), p 4649).

In process variant (a)(xiii), or (b) where X is CH₂OH and Y is -CH=N₂, the reaction is as described in den Hertzog et. al., recl.Trav. Chim. Pays-Bas, ( 1950),**69**, 700.

Reduction of A or B CO to CHOH can be readily accomplished using reducing agents well known to those skilled in the art, e.g. sodium borohydride in aqueous ethanol or lithium aluminiun hydride in ethereal solution.. This is analogous to methods described in EP 53964, US 384556 and J. Gutzwiller et. al. (1978) J.Amer.Chem.Soc., 100, 576.

The carbonyl group A or B may be reduced to CH₂ by treatment with a reducing agent such as hydrazine in ethylene glycol at 130-160°C in the presence of potassium hydroxide.

Reaction of a carbonyl group A or B with an organometallic reagent yields a group where R⁶ or R⁸ is OH and R⁷ or R⁹ is alkyl.

A hydroxy group A or B may be oxidised to a carbonyl group by oxidants well known to those skilled in the art , for example, manganese dioxide, pyridinium chlorochromate or pyridinium dichromate.

An A-B group COCH₂ may be converted to COCH-halogen. by treating the ketone or a derivative with a halogenating agent, reduced to CHOHCHCl and then converted to the epoxide which may in turn be reduced to CH₂CHOH.

Methods for conversion of CH=CH by reduction to CH₂CH₂ are well known to those skilled in the art, for example using hydrogenation over palladium on carbon as catalyst. Methods for conversion of CH=CH to give the A-B group as CHOHCH₂ or CH₂CHOH are well known to those skilled in the art for example by epoxidation and subsequenct reduction by metal hydrides, hydration, hydroboration or oxymercuration.

A hydroxyalkyl group A-B CH₂CHOH or CHOHCH₂ may be dehydrated to give the group CH=CH by treatment with an acid anhydride such as acetic anhydride.

An amide group CONHR^{11'} or NHR^{11'}CO may be reduced to the amine using a reducing agent such as lithium aluminium hydride

A ketone group may be converted to an amide CONH via the oxime by a Beckmann rearrangement (Ogliaruso, M.A.; Wolfe, J. F., *ibid.* pp 450-451; Beckwith, A. L. J., *ibid*. pp 131 ff.)

A hydroxy group A or B may be converted to azido by activation and displacement e.g. under Mitsunobu conditions using hydrazoic acid or by treatment with diphenylphosphorylazide and base, and the azido group in turn may be reduced to amino by hydrogenation.

A sulphur group A or B may be converted to the sulphoxide S(O)ₓ by oxidation with peracids or a wide range of oxidants known to those skilled in the art (see Advanced Organic Chemistry *(Ed. March, J.)* (John Wiley and Sons, 1985), p 1089 and refs. cited therein).

R^{1'}, R^{2'}, R^{3'} and R^{4'} are preferably R¹, R², R³ and R⁴. R^{1'} is preferably methoxy. R^{2'} is preferably hydrogen. R^{3'} is preferably R³ such as hydrogen, vinyl or a carboxy ester-containing group. R^{4'} is preferably R⁴, H or a protecting group.

Conversions of R^{1'}, R^{2'}, R^{3'} and R^{4'} and interconversions of R¹, R², R³ and R⁴ are conventional. In compounds which contain an optionally substituted hydroxy group, suitable conventional hydroxy protecting groups which may be removed without disrupting the remainder of the molecule include acyl and alkylsilyl groups.

For example R^{1'} methoxy is convertible to R^{1'} hydroxy by treatment with lithium and diphenylphosphine (general method described in Ireland et. al. (1973) J.Amer.Chem.Soc.,7829) or HBr. Alkylation of the hydroxy group with a suitable alkyl derivative bearing a leaving group such as halide and a protected amino, piperidyl, amidino or guanidino group or group convertible thereto, yields, after conversion/deprotection, R¹ (C₁₋₆) alkoxy substituted by optionally N-substituted amino, piperidyl, guanidino or amidino.

R^{3'} alkenyl is convertible to hydroxyalkyl by hydroboration using a suitable reagent such as 9-borabicyclo[3.3.1]nonane, epoxidation and reduction or oxymercuration.

R^{3'} carboxy ester groups may be converted to R³ hydroxymethyl by reduction with a suitable reducing agent such as lithium aluminium hydride.

R³ 1,2-dihydroxy can be prepared from R^{3'} alkenyl using osmium tetroxide or other reagents well known to those skilled in the art (see Advanced Organic Chemistry *(Ed. March, J.)* (John Wiley and Sons, 1985), p 732-737 and refs. cited therein) or epoxidation followed by hydrolysis (see Advanced Organic Chemistry *(Ed. March, J.)* (John Wiley and Sons, 1985), p 332,333 and refs. cited therein).

R³ vinyl can be chain extended by standard homologation e.g by conversion to hydroxyethyl followed by oxidation to the aldehyde which is then subjected to a Wittig reaction.

Compounds of formula (I) where R² and R³ are a divalent residue =CR^{5¹}R^{6¹} can be prepared by treatment of a compound of formula (I) where R³ is alken-1-yl with a strong base in an aprotic solvent. Suitable bases include Ph₂PLi/PhLi (as described in Ireland et. al., J. Amer. Chem .Soc. (1973), 7829), t-BuLi, and suitable solvents include THF and ether.

Substituents on R³ alkyl or alkenyl may be interconverted by conventional methods, for example hydroxy may be derivatised by esterification, acylation or etherification. Hydroxy groups may be converted to halogen, thiol, alkylthio, azido, alkylcarbonyl, amino, aminocarbonyl, oxo, alkylsulphonyl, alkenylsulphonyl or aminosulphonyl by conversion to a leaving group and substitution by the required group or oxidation as appropriate or reaction with an activated acid, isocyanate or alkoxyisocyanate. Primary and secondary hydroxy groups can be oxidised to an aldehyde or ketone respectively and alkyated with a suitable agent such as an organometallic reagent to give a secondary or tertiary alcohol as appropriate.

NH is converted to NR⁴ by conventional means such as alkylation with an alkyl halide in the presence of base, acylation/reduction or reductive alkylation with an aldehyde.

It will be appreciated that under certain circumstances interconvertions may interfere, for example, A or B hydroxy groups and the piperidine NH will require protection e.g. as a carboxy- or silyl-ester group for hydroxy and as an acyl derivative for piperidine nitrogen, during conversion of R^{1'}, R^{2'}, R^{3'} or R^{4'}.

Examples containing a *trans*-3,4-substituted piperidine ring may be prepared from the trans-3-vinyl-4-substituted piperidine prepared from the corresponding 3-vinyl-4-*cis*-isomer by the method of G. Engler *et al*. Helv. Chim. Acta **68**, 789-800 (1985); also described in Patent Application EP 0031753 (Pharmindustrie).

The method involves heating a 3-vinyl-4-alkyl-piperidine derivative of formula (VIII): 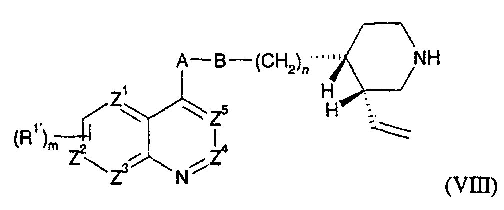 (prepared as an intermediate in the process of the invention) in dilute acid, preferably hydrochloric acid at pH 3.5, with 0.3-1.0 mol equivalents of formaldehyde. The main product of the reaction is the *trans*-isomer, which may be separated from the small quantity of *cis* isomer present, by conventional silica gel chromatography. It is convenient to convert the mixture of cis- and *trans*-piperidines (R^{4'} = H) to the tertiary amines of formula (I) by alkylation with an alkyl halide (preferably an iodide) in DMF in the presence of anhydrous potassium carbonate, prior to silica gel chromatography.

Compounds of formulae (IV), (V) and (Vb) are known compounds, (see for example Smith *et al, J. Amer. Chem. Soc.,* 1946, 68, 1301) or prepared analogously, see for example the references cited above for reaction variant (a).

A 4-chloroquinazoline is prepared from the corresponding quinazolin-4-one by reaction with phosphorus oxychloride (POCl₃) or phosphorus pentachloride, PCl₅. A quinazolinone and quinazolines may be prepared by standard routes as described by T.A. Williamson in Heterocyclic Compounds, **6**, 324 (1957) Ed. R.C. Elderfield. Pyridazines may be prepared by routes analogous to those described in Comprehensive Heterocyclic Chemistry, Volume 3, Ed A.J. Boulton and A. McKillop and napthyridines may be prepared by routes analogous to those described in Comprehensive Heterocyclic Chemistry, Volume 2, Ed A.J. Boulton and A. McKillop.

4-Hydroxy-1,5-naphthyridines can be prepared from 3-aminopyridine derivatives by reaction with diethyl ethoxymethylene malonate to produce the 4-hydroxy-3-carboxylic acid ester derivative with subsequent hydrolysis to the acid, followed by thermal decarboxylation in quinoline (as for example described for 4-Hydroxy-[1,5]naphthyridine-3-carboxylic acid, Joe T. Adams *et al., J.Amer.Chem.Soc*., 1946, **68**, 1317). A 4-hydroxy-[1,5]naphthyridine can be converted to the 4-chloro derivative by heating in phosphorus oxychloride. A 4-amino 1,5-naphthyridine can be obtained from the 4-chloro derivative by reaction with n-propylamine in pyridine. Similarly, 6-methoxy-1,5-naphthyridine derivatives can be prepared from 3-amino-6-methoxypyridine.

4-Methyl-1,5-naphthyridines can be prepared by methods well known to those skilled in the art (for example see H. Rapoport and A. D. Batcho, Journal of Organic Chemistry, 1963, 1753-1759). For example, nitrobenzene can be heated with oleum over a period of hours then water and a 3-aminopyridine added with heating. Slow addition of methyl vinyl ketone with heating produces the desired 4-methyl-1,5-naphthyridine.

1,5-Naphthyridines may be prepared by other methods well known to those skilled in the art (for examples see P.A. Lowe in "Comprehensive Heterocyclic Chemistry" Volume 2, p581-627, Ed A.R. Katritzky and C.W. Rees, Pergamon Press, Oxford, 1984).

The 4-hydroxy and 4-amino-cinnolines may be prepared following methods well known to those skilled in the art [see A.R. Osborn and K. Schofield, *J. Chem. Soc.* 2100 (1955)]. For example, a 2-aminoacetopheneone is diazotised with sodium nitrite and acid to produce the 4-hydroxycinnoline with conversion to chloro and amino derivatives as described for 1,5-naphthyridines. A 3-methyl substituent may be introduced by reaction of a 4-chlorocinnoline with lithium diisopropylamide at -75°C followed by alkylation with methyl iodide [see A. Turck et al. *Tetrahedron,* **47,** 13045 (1995)].

For compounds of formula (V) where Y is NHR^{11'} suitable amines may be prepared from the corresponding acid or alcohol (Y is CO₂H or CH₂OH). In a first instance, an N-protected piperidine containing an acid bearing substituent, can undergo a Curtius rearrangement and the intermediate isocyanate can be converted to a carbamate by reaction with an alcohol. Conversion to the amine may be achieved by standard methods well known to those skilled in the art used for amine protecting group removal. For example, an acid substituted N-protected piperidine such as t-butoxycarbonyl-protected 3-vinyl-4-piperidine acetic acid can undergo a Curtius rearrangement e.g. on treatment with diphenylphosphoryl azide and heating, and the intermediate isocyanate reacts in the presence of 2-trimethylsilylethanol to give the trimethylsilylethylcarbamate (T.L.Capson & C.D.Poulter, Tetrahedron Letters, 1984, 25, 3515). This undergoes cleavage on treatment with tetrabutylammonium fluoride to give the 4-piperidinemethylamine. Alternatively, an acid group (CH₂)ₙ₋₁CO₂H may be converted to (CH₂)ₙNHR₁₁ by reaction with an activating agent such as isobutyl chloroformate followed by an amine R^{11'}NH₂ and the resulting amide reduced with a reducing agent such as LiAlH₄.

In a second instance, an N-protected piperidine containing an alcohol bearing substituent undergoes a Mitsunobu reaction (for example as reviewed in Mitsunobu, Synthesis, (1981), 1), for example with succinimide in the presence of diethyl azodicarboxylate and triphenylphosphine to give the phthalimidoethylpiperidine. Removal of the phthaloyl group, for example by treatment with methylhydrazine, gives the amine of formula (V).

Compounds of formula (V) where n=1 or 2 may be prepared from the compound where n=0 by homologation eg starting from a compound of formula (V) where Y=CO₂H.

Conversions of R^{1'}, R^{2'}, R^{3'} and R^{4'} may be carried out on the intermediates of formulae (IV), (V) and (Vb) prior to their reaction to produce compounds of formula (I) in the same way as described above for conversions after their reaction.

Where a *trans*-substituted compound of formula (I) is required, a *trans*-substituted piperidine moiety of formula (V) may be prepared from the corresponding *cis* isomer of formula (V) having an R^{3'} vinyl group in the 3-position with a substituent that can subsequently be converted to the required group (CH₂)ₙY, for example CH₂CO₂R (where R is an alkyl group eg methyl or ethyl), by heating in formaldehyde.

The pharmaceutical compositions of the invention include those in a form adapted for oral, topical or parenteral use and may be used for the treatment of bacterial infection in mammals including humans.

The antibiotic compounds according to the invention may be formulated for administration in any convenient way for use in human or veterinary medicine, by analogy with other antibiotics.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine: tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) or a pharmaceutically acceptable salt or *in vivo* hydrolysable ester thereof is administered in the above-mentioned dosage range.

The compound of formula (I) may be the sole therapeutic agent in the compositions of the invention or a combination with other antibiotics or with a β-lactamase inhibitor may be employed.

Compounds of formula (I) are active against a wide range of organisms including both Gram-negative and Gram-positive organisms.

The following examples illustrate the preparation of certain compounds of formula (I) and the activity of certain compounds of formula (I) against various bacterial organisms.

### Example 1. [3R, 4S]-1-Heptyl-4-[N-methyl-N-(6-methoxy-quinazolin-4-yl)-2-aminoethyl]-3-ethenylpiperidine

### (a) [3R,4S]-3-Ethenyl-4-piperidineacetic acid

The title compound was prepared by modification of the procedure described by W.E. Daering and J.D. Chanley, *J. Amer. Chem. Soc*., 1946, **68,** 586 whereby quinone (225g, 0.70 mol) in t-butylalcohol (1l) and water (10 ml) was treated with potassium t-butoxide (170g, 1.5 mol). The mixture was stirred at 30C, while air was bubbled through the mixture for 3 days. The mixture was diluted with diethylether and water and the layers separated. The aqueous phase was extracted with ethyl acetate. The combined diethyl ether and ethyl acetate extracts were dried over magnesium sulfate and evaporated to give recovered starting material (97.6g, 43% recovery). The aqueous phase was acidified to pH5 with 5M hydrochloric acid. The precipitate was collected by filtration and washed with water and methanol. The filtrate contained [3R,4S]-3-Ethenyl-4-piperidineacetic acid.

### (b) [3R,4S]-1-(t-Butyloxycarbonyl)-3-ethenyl-4-piperidine acetic acid

Example 1a (96g, 0.57 mol) in dichloromethane (900 ml) and methanol (180 ml) was stirred while triethylamine (150 ml, 1.07 mol) were added. The mixture was stirred for 18h and evaporated. The residue was diluted with ethyl acetate and 2.5 m hydrochloric acid. The layers were separated and the aqueous phase extracted twice more with ethyl acetate. The combined organic extracts were extracted three times with sodium carbonate solution. The aqueous extracts were acidified to pH 2.5 with 5M hydrochloric acid and extracted with ethyl acetate three times. The combined organic extracts were washed with brine. dried over magnesium sulfate and evaporated to give the title compound as a buff solid (81.7g, 43%);
δ_{H} (CDCl₃, 250 MHz) 5.89-5.68 (1H, m), 5.23-5.06 (2H, m), 4.12 (1H, brs), 3.95 (2H, d, *J* 13Hz), 3.04 (1H, dd, *J* 3 and 9 Hz), 2.87 (1H, brs), 2.48-2.05 (4H, m), 1.63-1.35 (2H, m), and 1.45 (9H, s).

### (c) [3R, 4S]-1-Heptyl-4-(methylaminocarboxymethyl)-3-ethenylpiperidine

Example 1b (3g, 11.2 mmol) in ethyl acetate (50ml) was treated with 4-methylmorpholine (1.44 ml, 13.1 mmol) and isobutylchloroformate (1.8 ml, 13.9 mmol). After stirring for 1h the mixture was filtered and cooled in ice. Methylamine gas was bubbled through the mixture for 0.5h and the mixture stirred for a further 3h, then filtered, evaporated and purified on silica eluting with ethyl acetate to give a golden oil. Treatment of the oil with trifluoroacetic acid (9 ml) in dichloromethane (15 ml) for 1h, followed by evaporation and treatment with 1-iodoheptane (2.07 ml, 13.2 mmol) in dimethylformamide (15ml) in the presence of potassium carbonate (2.92g, 20.8 mmol) gave the title compound (2.61g), νₘₐₓ (film) 1647, 1558, and 1466 cm⁻¹.

### (d) [3R, 4S]-1-Heptyl-4-(2-methylaminoethyl)-3-ethenyl piperidine

The product from 1(c) (0.56g, 2.0 mmol) in tetrahydrofuran (15ml) was treated with lithium aluminium hydride (0.23g, 3 equiv) and heated at reflux for 3h. After allowing to cool the reaction was quenched with dilute sodium hydroxide, filtered through celite, and evaporated to give an oil (0.25g, 50%) MH⁺ 267.

### (e) Title compound.

The product from 1(d) (0.20g, 0.75 mmol) was heated with 4-chloro-6-methoxy-quinazoline (Smith *et al, J. Amer. Chem. Soc*., 1946, 68, 1301) (0.067g, 0.34 mmol) at 160°C for 18h. The mixture was cooled, diluted with dichloromethane, washed with sodium carbonate solution, dried and evaporated. Purification on silica gel eluting with methanol-chloroform-ammonia (5:95:0.5) gave a fawn oil (0.11g, 75%), MH⁺425

### Example 2. [3R, 4S]-1-Heptyl-4-[2-(6-methoxyquinazolin-4-oxy)ethyl]-3-ethenylpiperidine

### (a) Methyl [3R,4S]-3-ethenyl-4-piperidine acetate hydrochloride

Thionyl chloride (20ml) was added dropwise to a solution of Example 1b (10.0g, 37.1mmol) in methanol (400ml). The mixture was heated at reflux for 4 hours and then concentrated *in vacuo*. The residue was diluted with toluene and concentrated (x 3) to yield the title compound (8.10g, 100%).
δH (d₆ DMSO) 9.18 (1H, br s, exch.), 8.82 (1H, br s, exch), 6.03 (1H, m), 5.14 (2H, m), 3.59 (3H, s), 3.20-2.91 (4H, m), 2.62 (1H, m), 2.33-2.20 (3H, m), 1.80-1.52 (3H, m). MS (+ve ion electrospray) m/z 184 (MH⁺).

### (b) Methyl [3R,4S]-1-heptyl-3-ethenyl-4-piperidine acetate

Example 2a (8.1g, 36.9mmol) was dissolved in dry dimethylformamide (5ml) and n-heptyl bromide (0.172ml) and potassium carbonate (76mg) were added. The reaction mixture was heated to 80° with stirring for 2 hours, allowed to cool, diluted with water (10ml) and extracted with ethyl acetate (3 x 20ml). The combined organic extracts were dried over anhydrous magnesium sulphate, filtered and evaporated. The product was purified by chromatography on silica gel eluting with 50% ethyl acetate in hexane to give the title compound (9.71g, 94%).
δH (CDCl₃) 6.08 (1H, m), 5.03 (2H, m), 3.68 (3H, s), 2.79-2.62 (2H, m), 2.42-1.20 (20H, m), 0.89 (3H, t, *J* 6.7Hz). MS (+ve ion electrospray) m/z 282 (MH⁺).

### (c) [3R,4S]-1-Heptyl-3-ethenyl-4-(2-hydroxyethyl)-piperidine.

Lithium aluminium hydride (750mg, 19.7mmol) was added to a solution of Example 2b (2.5g, 9.8mmol) in tetrahydrofuran (100ml) at 0°C and the mixture stirred for 1 hour at that temperature. Water (0.75ml) was added, followed by 10% aqueous sodium hydroxide solution (1.1ml) and then water (1.9ml). The mixture was stirred for 1 hour and then filtered. The filtrate was concentrated *in vacuo* to yield the title compound as a orange oil (2.5g, 100%).
δH (CDCl₃) 6.12 (1H, m), 5.05 (2H, m), 3.67 (2H, t, *J* 6.5Hz), 2.76 (2H, m), 2.38-1.20 (21H, m), 0.89 (3H, t, *J* 6.8Hz). MS (+ve ion electrospray) m/z 254 (MH⁺).

### (d) Title compound

Example 2c (200 mg, 0.79 mmol) was dissolved in dry DMSO (3 ml) and treated with sodium (20 mg, 0.87 mmol) under argon. The reaction mixture was stirred for 2 hours at room temperature, treated with 4-chloro-6-methoxyquinazoline (Smith *et al, J. Amer. Chem. Soc*., 1946, 68, 1301) (154 mg, 0.79 mmol) and then heated at 60°C for 15 hours. The mixture was diluted with water and the product extracted with ethyl acetate (x3). The organic phase was dried over magnesium sulphate, filtered and concentrated *in vacuo* . The product was purified by chromatography on silica gel eluting with chloroform/methanol/35% ammonia solution (98:1.8:0.2) to yield the title compound (160 mg, 49%): MS (+ve ion electrospray) m/z 412 (MH⁺).

### Example 3. 1-Heptyl-4-(6-methoxy-1,5-naphthyridin-4-yl)aminocarbonyl piperidine

### (a) 1-Heptylpiperidine-4-carboxylic acid

### i) Benzyl 1-tert-butyloxycarbonylpiperidine-4-carboxylate

To a solution of 1-tert-butyloxycarbonylpiperidine-4-carboxylic acid (10 g, 43.7 mmol) and benzyl bromide (5.7 ml, 48 mmol) in dimethylformamide (100 ml) was added potassium carbonate (30.5 g, 222 mmol). This mixture was stirred for 12 h and ethyl acetate (100 ml) was added and washed successively with water (100 ml), brine (100 ml) and saturated sodium bicarbonate solution (100 ml). The organic phase was dried (MgSO₄), filtered and evaporated to give a colourless oil (10.39 g, 75%). MS m/z 320.

### ii) Benzyl piperidine-4-carboxylate

Benzyl 1-tert-butyloxycarbonylpiperidine-4-carboxylate (10.39 g, 32.6 mmol) was dissolved in dichloromethane (40 ml) and trifluoroacetic acid (40 ml) was added and the resulting mixture was stirred for 1 h. Evaporation of the solvents gave the title compound (12.37 g) as the trifluoroacetate salt in the form of an oil. MS m/z 220

### iii) Benzyl 1-heptylpiperidine-4-carboxylate

To a solution of benzyl piperidine-4-carboxylate (12.37g, 32.6 mmol) in dimethylformamide (100 ml) was added potassium carbonate (14.95 g, 107.6 mmol) and heptyl iodide (5.9 ml, 36 mmol) and the resulting solution was stirred for 12 h. Ethyl acetate (500 ml) was added and was washed with water (2 x 500 ml). The organic phase was dried (MgSO₄), filtered and evaporated to give an oil. Flash chromatography (silica gel, ethyl acetate as eluent) gave a colourless oil (6.58 g, 63%). MS m/z 318

### iv) Title compound

To a solution of Benzyl 1-heptylpiperidine-4-carboxylate (6.58 g, 20.8 mmol) in ethanol (100 ml) was added palladium supported on charcoal (10% palladium, 1.0 g). The mixture was treated with hydrogen (1 atmosphere) and allowed to stir for 12 h. Filtration and evaporation of the mixture gave a colourless solid (4.38 g, 93%). MS m/s (228)

### (b) 4-Hydroxy-6-methoxy-[1,5]naphthyridine-3-carboxylic acid ethyl ester

3-Amino-6-methoxypyridine (12.41 g) and diethyl ethoxymethylene malonate (20.2 ml) in Dowtherm A (400 ml) were heated at reflux, under argon for 1h. The cooled reaction mixture was poured onto pentane (1 litre). The precipitated solid was collected by filtration, washing with pentane. Drying afforded the title compound (24.78 g, crude).

### (c) 4-Hydroxy-6-methoxy-[1,5]naphthyridine-3-carboxylic acid

Example 3b (642 mg) in 10% aqueous sodium hydroxide (115 ml) was heated at reflux for 1.5h. The reaction mixture was cooled then acidified with glacial acetic acid. The preciptated solid was collected by filtration and washed with water. Drying *in vacuo* afforded the title compound as a beige solid (542 mg). m/z: 221 (MH⁺).

### (d) 4-Chloro-6-methoxy-[1,5]naphthyridine

Example 3c (6.82 g) was heated in quinoline (20 ml) at reflux for 2h, the mixture was cooled and poured into ether (200 ml) and the orange solid was filtered and washed with ether (5 x 200 ml). A sample (3.87 g) of the dried solid was treated with phosphorus oxychloride (30 ml) at room temp for 3h, the solvent was removed in vacuo and the residue quenched with crushed ice (200 g). The mixture was basified with ammonia solution and filtered. The solid was washed with DCM (10 x 100 ml) and which were combined and evaporated. Flash chromatography (SiO₂, DCM as eluent) gave the title compound as a yellow solid (3.00 g). m/z: 195,197 (MH⁺).

### (e) 4-Amino-6-methoxy-[1,5]naphthyridine

A solution of Example 3d (2.00 g) in pyridine (30 ml) was treated with n-propylamine hydrochloride (6 g) and the mixture heated at reflux for 16h. The reaction mixture was cooled and partitioned between water and ethyl acetate. The aqueous phase was washed with ethyl acetate, the combined organics dried (Na₂SO₄) and the solvent removed under reduced pressure. Purification by chromatography on silica gel eluting with 5-10% methanol in dichloromethane afforded the title compound as a yellow solid (1.00 g).
¹H NMR (CDCl₃) δ: 4.05 (3H, s), 5.36 (2H, bs), 6.71 (1H, d, J=5 Hz), 7.08 (1H, d, J=9Hz), 8.10 (1H, d, J=9Hz), 8.40 (1H, d, J=5Hz).
m/z: 176 (MH⁺).(f)Title compound

To a solution of Example 3a (114 mg), diisopropylamine (0.088 ml), and O-(7-azabenzotriazol-1-yl)-N,N,N',N'-tetramethyluronium hexafluorophosphate (HATU) (190 mg) in dimethylformamide (DMF) (1 ml) was added Example 3e (87.5 mg) in DMF (1 ml). The mixture was stirred for 18 h at room temperature. Evaporation of the solvent and aqueous work up with dichloromethane gave after flash chromatography on silica gel eluting with ethyl acetate-hexane the title compound as a colourless oil. The free base was treated with oxalic acid in ether and the resulting solid was collected, triturated with ether, to afford the oxalate salt as a colourless solid (150 mg).
MS (+ve ion electrospray) m/z 385 (MH+).

### Example 4. [3R, 4S]-1-Heptyl-3-ethenyl-4-N-(6-methoxy-1,5-naphthyridin-4-yl)-piperidineacetamide

### (a) [3R, 4R]-1-tert-Butyloxycarbonyl-3-ethenyl-N-(6-methoxy-1,5-naphthyridin-4-yl) piperidine-4-acetamide

Example 1b (538 mg) and Example 3e (350 mg) were coupled using the procedure of Example 3f.

### (b) [3R, 4R]-3-ethenyl-N-(6-methoxy-1,5-naphthyridin-4-yl) piperidine-4-acetamide

Treatment of the compound of Example 4a (850 mg) with trifluoroacetic acid (3 ml) and dichloromethane (DCM) (5 ml) for 1 h at room temperature gave, after evaporation, the trifluoroacetate salt of the amine. Addition of sodium bicarbonate solution to the residue and extraction with DCM gave the free base as a yellow oil (650 mg).

### (c) Title compound

The free base Example 4b (650 mg) was treated with heptaldehyde (0.42 mg) and methanol (10 ml), the resulting solution was stirred for 30 min before the addition of sodium triacetoxyborohydride (636 mg). The mixture was stirred at room temp for 4 h, evaporated and the residue subjected to aqueous work up with dichloromethane. The crude N-heptyl compound was chromatographed on silica gel eluting with methanol-dichloromethane to give the title compound as a colourless oil. The free base was treated with oxalic acid in ether and the resulting solid was collected, triturated with ether, to afford the oxalate salt as a colourless solid (391 mg).
MS (+ve ion electrospray) m/z 425 (MH+).

### Example 5. [3R,4S]-1-Heptyl-3-ethenyl-4-[2-(R,S)-hydroxy-3-(6-methoxy-1,5-naphthyridin-4-yl)propyl]piperidine

### (a) 6-Methoxy-4-methyl-1,5-naphthyridine

5-Amino-2-methoxypyridine (12.5 g, 100 mmol) and iron(III) chloride hexahydrate were stirred at 70 °C in acetic acid (320 ml). Methyl vinyl ketone (8.8 ml, 105 mmol) was added dropwise over 10 min. The mixture was stirred at this temperature for 2 h then cooled to room temperature. The acetic acid was evaporated and the residue was treated with sodium hydroxide solution (40% aqueous, 200 ml). The mixture was then extracted with EtOAc (5 x 400 ml), the combined extracts were then evaporated and chromatography (SiO₂, 50% EtOAc/hexane to 100% EtOAc) gave a dark brown solid (5.54 g, 32%). LCMS m/z 175.

### (b) N-Methyl-N-methoxy-[3R,4S]-1-(t-butyloxycarbonyl)-3-ethenyl-4-piperidine acetamide

To a solution of the acid of Example 1b (10 g, 37 mmol) in dimethylformamide (80 ml) was added, sequentially. diisopropylethylamine (74 mmol, 12.9 ml), N-methoxymethylamine hydrochloride (37 mmol, 3.61 g) and O-(7-azabenzotriazol-1-yl)-N,N,N',N'-tetramethyluronium hexafluorophosphate (37 mmol, 14.07 g). The mixture was stirred at room temperature overnight and the solvent was then evaporated. The residue was dissolved in EtOAc (100 ml) and washed with water (100 ml), saturated sodium bicarbonate solution (100 ml) and brine (100 ml). The solvent was removed and the residue (11g, 95%) used in the next step. LCMS m/z 313

### (c) N-Methyl-N-methoxy-[3R,4S]-3-ethenyl-4-piperidine acetamide

The acetamide of Example 5b (11 g, 35 mmol) was dissolved in dichloromethane (50 ml) and trifluoroacetic acid (30 ml) added. The mixture was stirred at room temperature for 1 h and then evaporated. The residue was treated with sodium bicarbonate solution (saturated, 100 ml) and dichloromethane (100 ml). The mixture was stirred rapidly for 5 min and the layers separated. The aqueous layer was washed with dichloromethane (100 ml) and neutralised (pH 7) and further washed with DCM (50 ml). The aqueous layer was treated with aqueous sodium hydroxide solution (pH 14) and further extracted with dichloromethane (2 x 50 ml) and ethyl acetate (150 ml). The combined organic extracts were evaporated to give the title compound as an orange oil (6 g, 81%). LCMS m/z 213

### (d) N-Methyl-N-methoxy-[3R,4S]-1-heptyl-3-ethenyl-4-piperidine acetamide

The acetamide of Example 5c (6 g, 28.3 mmol) was dissolved in methanol (50 ml) and heptaldehyde (40 mmol, 5.6 ml) added and the mixture stirred for 1 h. Sodium triacetoxyborohydride (45 mmol, 9.54 g) was added and the mixture stirred for 3 h. The solvent was evaporated and the residue treated with sodium bicarbonate solution (saturated, 100 ml) and dichloromethane (100 ml). The mixture was stirred rapidly for 5 min and the layers separated. The organic layer was washed with brine (50 ml) and then evaporated. The residue was chromatographed (SiO₂, EtOAc to 20% methanol/EtOAc) to give a dark brown oil (6.48 g, 74%). LC MS m/z 311

### (e) N-Methyl-N-methoxy-[3R,4S]-1-heptyl-3-ethenyl-4-piperidine acetaldehyde

The acetamide of Example 5d (6.48 g, 20.9 mmol) in tetrahydrofuran (30 ml) was added dropwise to a solution of lithium aluminium hydride (23 mmol, 1M in ether, 23 ml) in ether (50 ml) at -50 °C over 15 min. After a further 10 min the temperature was raised to 5 °C and cooled immediately to -50 °C again. A solution of NaHSO₄ (5 g in 50 ml) was added dropwise and the temperature allowed to warm to ambient. The mixture was stirred for a further 1 h and then filtered through celite. Chromatography (SiO₂, EtOAc) gave an orange oil (1.2 g, 23 %). LCMS m/z 252

### (f) Title compound

Lithium diisopropylamide (2.1 mmol, 1.5 M, 1.4 ml) was added to cooled tetrahydrofuran (2 ml) under argon at -78 °C. A solution of Example 5a (2 mmol, 348 mg) in tetrahydrofuran (3 ml) was added dropwise and the mixture stirred for 30 min. The acetaldehyde Example 5e(2 mmol, 536 mg) in tetrahydrofuran (3 ml) was then added dropwise and the mixture stirred for 30 min at -78 °C and 3 h at room temperature. acetic acid (5 drops) was then added followed by scavenger resin (polymer- tosylhydrazine, 2.42 mmol/g, 3 equivalents, 6 mmol, 2.5 g). The mixture was then stirred for 1 h. The resin was filtered and washed with dichloromethane (20 ml) and the organic solvents evaporated. Chromatography (SiO₂, dichloromethane to 2% methanol/ dichloromethane) gave a yellow oil (300 mg, 35%). LCMS m/z 426.

### Example 6. [3R,4S]-1-Heptyl-4-N-(6-methoxy-1,5-naphthyridin-4-yl)-3,4-piperidinediacetamide oxalate

### (a) [3R,4S]-1-Benzyloxycarbonyl-3-ethenyl-4-piperidineacetic acid, methyl ester

Benzyl chloroformate (8.34ml, 58.2 mmol) was added dropwise to a solution of Example 2a (9.70g, 52.4 mmol) and triethylamine (8.15ml, 58.2 mmol) in dry dichloromethane (100ml) with cooling. After overnight stirring, the mixture was washed with dilute hydrochloric acid and water, dried and evaporated. Chromatography on silica gel (1:1 ethyl acetate/petrol) gave the title compound (10.91g, 66%).
MS (+ve ion electrospray) m/z 318 (MH+)

### (b) [3R,4S]-1-Benzyloxycarbonyl-3-(2-hydroxyethyl)-4-piperidineacetic acid, methyl ester.

A solution of Example 6a (9.75g, 30.7 mmol) in dry tetrahydrofuran (200ml) was treated with 9-borabicyclo[3.3.1]nonane (0.5M in hexane, 134.5ml, 67.25 mmol) and stirred overnight at room temperature. The mixture was then cooled in ice and treated with 2M aqueous sodium hydroxide (42ml) followed by slow addition of 30% hydrogen peroxide (28ml). After stirring for 1.5 hours, ethyl acetate and water were added and the phases separated. The aqueous phase was re-extracted (ethyl acetate) and the combined organics were washed with water and brine, dried and evaporated. Chromatography on silica gel (2-5% methanol/dichloromethane) gave the title compound (4.55g, 44%).
MS (+ve ion electrospray) m/z 336 (MH+)

### (c) [3R,4S]-1-Benzyloxycarbonyl-3,4-piperidinediacetic acid, 3-tert-butyl-4-methyl diester.

To a solution of pyridinium dichromate (10.46g, 27.3 mmol) in dichloromethane (130ml) and dimethylformamide (45ml) were added tert-butanol (38.7ml, 409.5 mmol) and acetic anhydride (12.97ml, 136.5 mmol). A solution of Example 6b (4.55g, 13.65 mmol) in dichloromethane (50ml) was added and the mixture was stirred for 72 hours. The mixture was treated with ethanol and solid sodium bicarbonate, stirred for 1hour, then filtered and evaporated. The residue was dissolved in ethyl acetate and water, filtered under suction and the phases were separated. The organic phase was washed with water, dried and evaporated. Chromatography on silica gel (50-60% ether/petrol) gave the title compound (2.10g, 38%).
MS (+ve ion electrospray) m/z 406 (MH+)

### (d) [3R,4S]-1-Heptyl-3,4-piperidinediacetic acid, 3-tert-butyl-4-methyl diester.

A solution of Example 6c (1.9g, 4.65 mmol) and heptanal (0.67ml, 4.8mmol) in methanol (50 ml) was hydrogenated over 10% palladium/charcoal (0.5g) at 10 psi for 16 hours. The mixture was filtered and evaporated to give the title compound (1.61g, 94%). MS (+ve ion electrospray) m/z 370 (MH+)

### (e) [3R,4S]-1-Heptyl-3,4-piperidinediacetic acid, 3-tert-butyl ester.

A solution of Example 6d (1.79g, 3.82 mmol) in 1,4-dioxane (76ml) was treated with 2M sodium hydroxide (3.8ml, 7.6 mmol) and stirred for 72 hours. The mixture was filtered and evaporated, and the residue was partitioned between water and ethyl acetate. The aqueous phase was neuralised to pH7, saturated with sodium chloride and extracted with methanol/ethyl acetate. The extracts were dried and evaporated to give the title compound (1.01g, 74%).
MS (+ve ion electrospray) m/z 356 (MH+)

### (f) [3R,4S]-1-Heptyl-3-(tert-butoxycarbonylmethyl)-4-N-(6-methoxy-[1,5]-naphthyridin-4-yl)-4-piperidineacetamide

Example 6e (0.5g, 1.3 mmol) in dry dichloromethane (10ml) was treated with dimethyl formamide (1 drop) and oxalyl chloride (0.65ml, 7.5 mmol). After stirring for 4 hours, the mixture was evaporated to dryness. The residue was dissolved in toluene, re-evaporated, then dissolved in dry dimethylformamide (10ml). To this solution was added Example 3e (0.34g, 1.9 mmol), 4-dimethylaminopyridine (20mg) and triethylamine (0.26ml). After stirring for 72 hours, the mixture was evaporated and the residue was dissolved in ethyl acetate, washed with water, dried and evaporated to give the title compound (0.38g, 57%).
MS (+ve ion electrospray) m/z 513 (MH+)

### (g) [3R,4S]-1-Heptyl-3-carboxymethyl-4-N-(6-methoxy-[1,5]-naphthyridin-4-yl)-4-piperidineacetamide

Example 6f (0.35g, 0.68mmol) was dissolved in 2M hydrochloric acid (2ml) with gentle warming and the solution was allowed to stand for 2 hours at room temperature. The solution was then neutralised with sodium bicarbonate and extracted with ethyl acetate/methanol. The extract was extracted three times with sodium bicarbonate, and this extract was neutralised and extracted several times with ethyl acetate/methanol. The extract was dried and evaporated to give the title compound (0.1g). Evaporation of the aqueous solution to dryness and further extraction of the residue with dichloromethane/methanol gave more title compound (0.13g; total 0.23g, 74%).
MS (+ve ion electrospray) m/z 457 (MH+)

### (h) Title compound

Example 6g (0.06g, 0.13mmol) in dry dichloromethane (2.5ml) was treated with oxalyl chloride (0.055ml, 0.66 mmol) and dimethyl formamide (1 drop) and the mixture was stirred, with further additions of oxalyl chloride (0.03ml) until acid chloride formation was complete. The mixture was evaporated and the residue was dissolved in toluene , re-evaporated and dissolved in dry dimethyl formamide (2.5ml). Ammonia was bubbled through the solution for 15 minutes, then the mixture was stirred for 2 hours and evaporated. The residue was extracted with ethyl acetate /methanol, washed with brine, dried and evaporated. Chromatography on silica gel (5-10% methanol/dichloromethane) gave the free base of the title compound (46%).
¹H NMR (CDCl₃); δ 0.88(3H, t, J=7), 1.28(9H, m), 1.62(2H, m), 1.84(2H, m), 2.25(1H, dd, J=13,4), 2,3-2.6(7H, m), 2.63(1H, m), 2.85-3.25(2H, m), 4.13(3H, s), 5.46(1H, s), 6.85(1H, broad), 7.17(1H, d, J=9), 8.21(1H, d, J=9), 8.48(1H, d, J=5), 8.69(1H, d, J=5), 9.39(1H, s).
MS (+ve ion electrospray) m/z 456 (MH+)

The free base was treated with 0.1M oxalic acid in ether (1.1ml) to give the title compound.

The following compounds were prepared by analogous methods to those described herein.

### Example 7. [3R,4S]-1-Heptyl-4-N-(6-methoxy-1,5-naphthyridin-4-yl)-3-(1-(R,S)-2-dihydroxyethyl)-piperidineacetamide oxalate

### Example 8. [3R, 4S]-1-Heptyl-3-ethenyl-4-N-(6-methoxy-cinnolin-4-yl)-piperidineacetamide

### Biological Activity

The MIC (µg/ml) of compounds 1 and 2 against various organisms was determined .

| Organism | 1 | 2 |
|---|---|---|
| **S. aureus Oxford** | 0.5 | 4 |
| **S. aureus WCUH29** | 0.5 | 4 |
| **S. aureus Carter 37** | 0.5 | 4 |
| **E. faecalis 1** | 4 | 8 |
| **M. catarrhalis Ravasio** | 0.5 | 4 |
| **S. pneumoniae R6** | 0.5 | 8 |

Examples 3-6 had MIC values less than or equal to 1 against some of these organisms.

## Claims

1. A compound of formula (I) or a pharmaceutically acceptable derivative thereof: 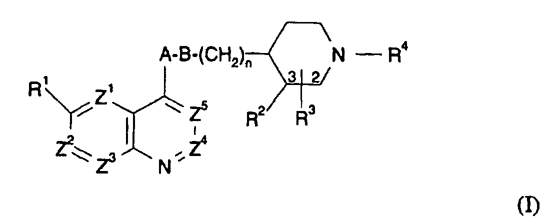 wherein:
one of Z¹, Z², Z³, Z⁴ and Z⁵ is N and the remainder are CH;
R¹ is hydrogen, hydroxy; (C₁₋₆) alkoxy optionally substituted by (C₁₋₆)alkoxy, amino, piperidyl, guanidino or amidino optionally N-substituted by one or two (C₁₋₆)alkyl, acyl or (C₁₋₆)alkylsulphonyl groups, NH₂CO, hydroxy, thiol, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted (C₁₋₆)alkyl; halogen; (C₁₋₆)alkyl; (C₁₋₆)alkylthio; nitro; trifluoromethyl; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups;
either R² is hydrogen; and
R³ is in the 2- or 3-position and is hydrogen or (C₁₋₆)alkyl or (C₂₋₆)alkenyl optionally substituted with 1 to 3 groups selected from:
thiol; halogen; (C₁₋₆)alkylthio; trifluoromethyl; azido; (C₁₋₆)alkoxycarbonyl; (C₁₋₆)alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; amino optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl; aminocarbonyl wherein the amino group is optionally mono- or disubstituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl or (C₂₋₆)alkenylcarbonyl; oxo; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₂₋₆)alkenyl; or
R³ is in the 3-position and R² and R³ together are a divalent residue =CR^{5¹}R^{6¹} where R^{5¹} and R^{6¹} are independently selected from H, (C₁₋₆)alkyl, (C₂₋₆)alkenyl, aryl(C₁₋₆)alkyl and aryl(C₂₋₆)alkenyl, any alkyl or alkenyl moiety being optionally substituted by 1 to 3 groups selected from those listed above for substituents on R³;
R⁴ is a group -CH₂-R⁵ in which R⁵ is selected from:
(C₃₋₁₂)alkyl; hydroxy(C₃₋₁₂)alkyl; (C₁₋₁₂)alkoxy(C₃₋₁₂)alkyl; (C₁₋₁₂)alkanoyloxy(C₃₋₁₂)alkyl; (C₃₋₆)cycloalkyl(C₃₋₁₂)alkyl; hydroxy-, (C₁₋₁₂)alkoxy- or (C₁₋₁₂)alkanoyloxy-(C₃₋₆)cycloalkyl(C₃₋₁₂)alkyl; cyano(C₃₋₁₂)alkyl; (C₂₋₁₂)alkenyl; (C₂₋₁₂)alkynyl; tetrahydrofuryl; mono- or di-(C₁₋₁₂)alkylamino(C₃₋₁₂)alkyl; acylamino(C₃₋₁₂)alkyl; (C₁₋₁₂)alkyl- or acylaminocarbonyl(C₃₋₁₂)alkyl; mono- ordi- (C₁₋₁₂)alkylamino(hydroxy) (C₃₋₁₂)alkyl; optionally substituted phenyl(C₁₋₂)alkyl, phenoxy(C₁₋₂)alkyl or phenyl(hydroxy)(C₁₋₂)alkyl; optionally substituted diphenyl(C₁₋₂)alkyl; optionally substituted phenyl(C₂₋₃)alkenyl; optionally substituted benzoyl or benzoylmethyl; optionally substituted heteroaryl(C₁₋₂)alkyl;and optionally substituted heteroaroyl or heteroaroylmethyl;
n is 0, 1 or 2;
A is NR¹¹, O, S(O)ₓ or CR⁶R⁷ and B is NR¹¹, O, S(O)ₓ or CR⁸R⁹ where x is 0, 1 or 2 and wherein:
each of R⁶ and R⁷, R⁸ and R⁹ is independently selected from: H; thiol; (C₁₋₆)alkylthio; halo; trifluoromethyl; azido; (C₁₋₆)alkyl; (C₂₋₆)alkenyl; (C₁₋₆)alkoxycarbonyl; (C₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₁₋₆)alkenyl;
or R⁶ and R⁸ together represent a bond and R⁷ and R⁹ are as above defined;
or R⁶ and R⁸ together represent -O- and R⁷ and R⁹ are both hydrogen;
or R⁶ and R⁷ or R⁸ and R⁹ together represent oxo;
and each R¹¹ is independently H, trifluoromethyl, (C₁₋₆)alkyl, (C₁₋₆)alkenyl, (C₁₋₆)alkoxycarbonyl, (C₁₋₆)alkylcarbonyl, aminocarbonyl wherein the amino group is optionally mono- or di-substituted by (C₁₋₆)alkoxycarbonyl, (C₁₋₆)alkylcarbonyl, (C₁₋₆)alkenyloxycarbonyl, (C₂₋₆)alkenylcarbonyl, (C₁₋₆)alkyl or (C₁₋₆)alkenyl;
provided that A and B cannot both be selected from NR¹¹, O and S(O)ₓ and when one of A and B is CO the other is not CO, O or S(O)ₓ;
wherein
'heterocyclic' is an optionally substituted aromatic or non-aromatic, single or fused, ring containing up to four hetero-atoms in each ring selected from oxygen, nitrogen and sulphur, and having from 4 to 7 ring atoms, which rings may be unsubstituted or substituted by up to three groups selected from amino, halogen, (C₁₋₆)alkyl, (C₁₋₆)alkoxy, halo(C₁₋₆)alkyl, hydroxy, carboxy, carboxy salts, (C₁₋₆)alkoxycarbonyl, (C₁₋₆)alkoxycarbonyl(C₁₋₆)alkyl, aryl, and oxo groups, and wherein any amino group forming part of a single or fused non-aromatic heterocyclic ring as defined above is optionally substituted by (C₁₋₆)alkyl optionally substituted by hydroxy, (C₁₋₆)alkoxy, thiol, (C₁₋₆)alkylthio, halo or trifluoromethyl, acyl and (C₁₋₆)alkylsulphony) groups;
'aryl' is optionally substituted phenyl or naphthyl
optionally substituted phenyl or naphthyl is optionally substituted with up to five groups selected from halogen, mercapto, (C₁₋₆)alkyl, phenyl, (C₁₋₆)alkoxy, hydroxy(C₁₋₆)alkyl, mercapto (C₁₋₆)alkyl, halo(C₁₋₆)alkyl, hydroxy, amino, nitro, carboxy, (C₁₋₆)alkylcarbonyloxy, (C₁₋₆)alkoxycarbonyl, formyl and (C₁₋₆)alkylcarbonyl groups;
'acyl' is (C₁₋₆)alkoxycarbonyl, formyl or (C₁₋₆) alkylcarbonyl.

2. A compound according to claim 1 wherein Z¹ is N and Z²-Z⁵ are each CH or Z⁵ is N and Z¹-2⁴ are each CH.

3. A compound according to claim 1 or 2 wherein R¹ is methoxy, amino(C₃₋₅)alkyloxy, guanidino(C₃₋₅)alkyloxy or fluoro, most preferably methoxy.

4. A compound according to any preceding claim wherein R³ is in the 3-position and is arainocarbonyl(C₁₋₆)alkyl, hydroxy(C₁₋₆)alkyl or 1,2-dihydroxy(C₂₋₆)alkyl optionally substituted on the hydroxy group(s).

5. A compound according to any preceding claim wherein AB is NHCO, NHCOCH₂ or CH₂CH(OH)CH₂.

6. A compound according to any preceding claim wherein R⁴ is (C₅₋₁₀)alkyl, unsubstituted phenyl(C₂₋₃)alkyl or unsubstituted phenyl(C₃₋₄)alkenyl.

7. A compound according to claim 1 selected from:
[3R, 4S]-1-Heptyl-4-[N-methyl-N-(6-methoxy-quinazolin-4-yl)-2-aminoethy)]-3-ethenylpiperidine;
[3R, 4S]-1-Heptyl-4-[2-(6-methoxyquinazolin-4-oxy)ethyl]-3-ethenylpiperidine;
1-Heptyl-4-(6-methoxy-1,5-naphthyridin-4-yl)aminocarbonyl piperidine;
[3R, 4S]-1-Heptyl-3-ethenyl-4-N-(6-methoxy-1,5-naphthyridin-4-yl)-piperidineacetamide;
[3R,4S]-1-Heptyl-3-ethenyl-4-[2-(R,S)-hydroxy-3-(6-methoxy-1,5-naphthyridin-4-yl)propyl]piperidine;
[3R,4S]-1-Heptyl-4-N-(6-methoxy-1,5-naphthyridin-4-yl)-3,4-piperidinediacetamide;
[3R,4S]-1-Heptyl-4-N-(6-methoxy-1,5-naphthyridin-4-yl)-3-(1-(R,S)-2-dihydroxyethyl)-piperidineacetamide;
[3R, 4S]-1-Heptyl-3-ethenyl-4-N-(6-methoxy-cinnolin-4-yl)-piperidineacetamide, or a pharmaceutically acceptable derivative of any of the foregoing compounds.

8. A process for preparing compounds of formula (I), or a pharmaceutically acceptable derivative thereof according to claim 1, which process comprises:
(a) reacting a compound of formula (IV) with a compound of formula (V): 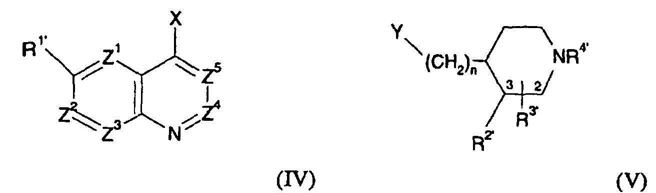 wherein Z¹, Z², Z³, Z⁴ and Z⁵, m, n, R¹, R², R³ and R⁴ are as defined in formula (I), and X and Y may be the following combinations:
(i) X is M and Y is CH₂CO₂R^{x}
(ii) X is CO₂R^{y} and Y is CH₂CO₂R^{x}
(iii) one of X and Y is CH=SPh₂ and the other is CHO
(iv) X is CH₃ and Y is CHO
(v) X is CH₃ and Y is CO₂R^{x}
(vi) X is CH₂CO₂R^{y} and Y is CO₂R^{x}
(vii) X is CH=PR^{z}₃ and Y is CHO
(viii) X is CHO and Y is CH=PR^{z}₃
(ix) X is halogen and Y is CH=CH₂
(x) one of X and Y is COW and the other is NHR^{11'}
(xi) one of X and Y is (CH₂)ₚ-V and the other is (CH₂)_{q}NHR^{11'}, (CH₂)_{q}OH, (CH₂)_{q}SH or (CH₂)_{q}SCOR^{x} where p+q=1
(xii) one of X and Y is CHO and the other is NHR^{11'}
(xiii) one of X and Y is OH and the other is -CH=N₂
in which V and W are leaving groups, R^{x} and R^{y} are (C₁₋₆)alkyl and R^{z} is aryl or (C₁₋₆)alkyl;
or
(b) reacting a compound of formula (IV) with a compound of formula (Vb): 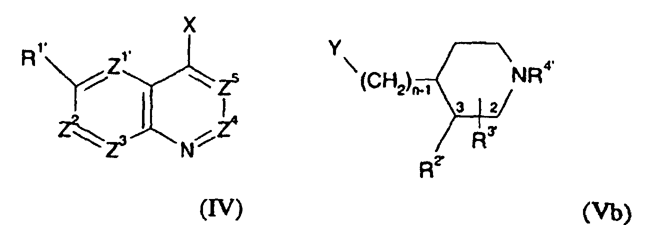 wherein Z¹, Z², Z³, Z⁴ and Z⁵, m, n, R¹, R², R³ and R⁴ are as defined in formula (I), X is CH₂NHR^{11'} and Y is CHO or COW or X is CH₂OH and Y is -CH=N₂;
in which R^{11'}, R^{1'}, R^{2'}, R^{3'} and R^{4'} are R¹¹, R¹, R², R³ and R⁴ or groups convertible thereto, and thereafter optionally or as necessary converting R^{11'}, R^{1'}, R^{2'}, R^{3'} and R^{4'} to R¹¹, R¹, R², R³ and R⁴, converting A-B to other A-B, interconverting R¹¹, R¹, R², R³ and/or R⁴ and forming a pharmaceutically acceptable derivative thereof.

9. A pharmaceutical composition comprising a compound of formula (I) or a pharmaceutically acceptable derivative thereof according to claim 1, and a pharmaceutically acceptable carrier.

10. The use of a compound of formula (I) or a pharmaceutically acceptable derivative thereof according to claim 1 in the manufacture of a medicament for use in the treatment of bacterial infections in mammals.

## Patentansprüche

1. Verbindung der Formel (I) oder ein pharmazeutisch verträgliches Derivat davon: 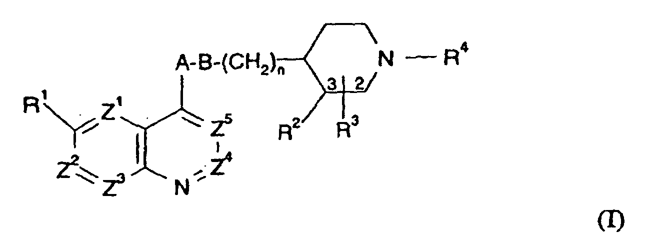 wobei:
eines von Z¹, Z², Z³, Z⁴ und Z⁵ N ist und die übrigen CH sind;
R¹ Wasserstoff, Hydroxy; (C₁₋₆)Alkoxy, das gegebenenfalls mit (C₁₋₆)Alkoxy, Amino, Piperidyl, Guanidino oder Amidino, gegebenenfalls N-substituiert mit einem oder zwei (C₁₋₆)Alkyl-, Acyl- oder (C₁₋₆)Alkylsulfonylresten, NH₂CO, Hydroxy, Thiol, (C₁₋₆)Alkylthio, Heterocyclylthio, Heterocyclyloxy, Arylthio, Aryloxy, Acylthio, Acyloxy oder (C₁₋₆)Alkylsulfonyloxy substituiert ist; (C₁₋₆)Alkoxy-substituiertes (C₁₋₆)Alkyl; Halogen; (C₁₋₆)Alkyl; (C₁₋₆)Alkylthio; Nitro; Trifluormethyl; Azido; Acyl; Acyloxy; Acylthio; (C₁₋₆)Alkylsulfonyl; (C₁₋₆)Alkylsulfoxid; Arylsulfonyl; Arylsulfoxid oder eine Amino-, Piperidyl-, Guanidino- oder Amidinogruppe, die gegebenenfalls mit einem oder zwei (C₁₋₆)Alkyl-, Acyl- oder (C₁₋₆)Alkylsulfonylresten N-substituiert sind, ist;
entweder
R² Wasserstoff ist; und
R³ in der 2- oder 3-Position ist und Wasserstoff oder (C₁₋₆)Alkyl oder (C₂₋₆)Alkenyl ist, die gegebenenfalls mit 1 bis 3 Resten substituiert sind, ausgewählt aus Thiol; Halogen; (C₁₋₆)Alkylthio; Trifluormethyl; Azido; (C₁₋₆)Alkoxycarbonyl; (C₁₋₆)Alkylcarbonyl; (C₂₋₆)Alkenyloxycarbonyl; (C₂₋₆)Alkenylcarbonyl; Hydroxy, das gegebenenfalls mit (C₁₋₆)Alkyl, (C₂₋₆)Alkenyl, (C₁₋₆)Alkoxycarbonyl, (C₁₋₆)Alkylcarbonyl, (C₂₋₆)Alkenyloxycarbonyl, (C₂₋₆)Alkenylcarbonyl oder Aminocarbonyl, wobei die Aminogruppe gegebenenfalls mit (C₁₋₆)Alkyl, (C₂₋₆)Alkenyl, (C₁₋₆)Alkylcarbonyl oder (C₂₋₆)Alkenylcarbonyl substituiert ist, substituiert ist; Amino, das gegebenenfalls mit (C₁₋₆)Alkoxycarbonyl, (C₁₋₆)Alkylcarbonyl, (C₂₋₆)Alkenyloxycarbonyl, (C₂₋₆)Alkenylcarbonyl, (C₁₋₆)Alkyl, (C₂₋₆)Alkenyl, (C₁₋₆)Alkylsulfonyl, (C₂₋₆)Alkenylsulfonyl oder Aminocarbonyl, wobei die Aminogruppe gegebenenfalls mit (C₁₋₆)Alkyl oder (C₂₋₆)Alkenyl substituiert ist, mono- oder disubstituiert ist; Aminocarbonyl, wobei die Aminogruppe gegebenenfalls mit (C₁₋₆)Alkyl, (C₂₋₆)Alkenyl, (C₁₋₆)Alkoxycarbonyl, (C₁₋₆)Alkylcarbonyl, (C₂₋₆)Alkenyloxycarbonyl oder (C₂₋₆)Alkenylcarbonyl mono- oder disubstituiert ist; Oxo; (C₁₋₆)Alkylsulfonyl; (C₂₋₆)Alkenylsulfonyl; oder (C₁₋₆)Aminosulfonyl, wobei die Aminogruppe gegebenenfalls mit (C₁₋₆)Alkyl oder (C₂₋₆)Alkenyl substituiert ist;
oder
R³ in der 3-Position ist und R² und R³ zusammen ein zweiwertiger Rest =CR^{5¹}R^{6¹} sind, wobei R^{5¹} und R^{6¹} unabhängig aus H, (C₁₋₆)Alkyl, (C₂₋₆)Alkenyl, Aryl(C₁₋₆)alkyl und Aryl(C₂₋₆)alkenyl ausgewählt sind, wobei jede Alkyl- oder Alkenyleinheit gegebenenfalls mit einem bis drei Resten, ausgewählt aus denen, die für Substituenten an R³ vorstehend aufgeführt sind, substituiert ist;
R⁴ ein Rest -CH₂-R⁵ ist, wobei R⁵ aus:
(C₃₋₁₂)Alkyl; Hydroxy(C₃₋₁₂)alkyl; (C₁₋₁₂)Alkoxy(C₃₋₁₂)alkyl; (C₁₋₁₂)Alkanoyloxy-(C₃₋₁₂)alkyl; (C₃₋₆)Cycloalkyl(C₃₋₁₂)alkyl; Hydroxy-, (C₁₋₁₂)Alkoxy- oder (C₁₋₁₂)Alkanoyloxy-(C₃₋₆)cycloalkyl(C₃₋₁₂)alkyl; Cyano(C₃₋₁₂)alkyl; (C₂₋₁₂)Alkenyl; (C₂₋₁₂)Alkinyl; Tetrahydrofuryl; Mono- oder Di-(C₁₋₁₂)alkylamino(C₃₋₁₂)alkyl; Acylamino(C₃₋₁₂)alkyl; (C₁₋₁₂)Alkyl- oder Acylaminocarbonyl(C₃₋₁₂)alkyl; Mono- oder Di-(C₁₋₁₂)alkylamino(hydroxy)(C₃₋₁₂)alkyl; gegebenenfalls substituiertem Phenyl(C₁₋₂)alkyl, Phenoxy(C₁₋₂)alkyl oder Phenyl(hydroxy)(C₁₋₂)alkyl; gegebenenfalls substituiertem Diphenyl(C₁₋₂)alkyl; gegebenenfalls substituiertem Phenyl(C₂₋₃)alkenyl; gegebenenfalls substituiertem Benzoyl oder Benzoylmethyl; gegebenenfalls substituiertem Heteroaryl(C₁₋₂)alkyl; und gegebenenfalls substituiertem Heteroaroyl oder Heteroaroylmethyl ausgewählt ist;
n 0, 1 oder 2 ist;
A NR¹¹, O, S(O)ₓ oder CR⁶R⁷ ist und B NR¹¹, O, S(O)ₓ oder CR⁸R⁹ ist, wobei x 0, 1 oder 2 ist und wobei:
jeder Rest R⁶ und R⁷, R⁸ und R⁹ unabhängig aus: H; Thiol; (C₁₋₆)Alkylthio; Halogen; Trifluormethyl; Azido; (C₁₋₆)Alkyl; (C₂₋₆)Alkenyl; (C₁₋₆)Alkoxycarbonyl; (C₁₋₆)Alkylcarbonyl; (C₂₋₆)Alkenyloxycarbonyl; (C₂₋₆)Alkenylcarbonyl; Hydroxy, Amino oder Aminocarbonyl, die gegebenenfalls wie für die entsprechenden Substituenten in R³ substituiert sind; (C₁₋₆)Alkylsulfonyl; (C₂₋₆)Alkenylsulfonyl; oder (C₁₋₆)Aminosulfonyl, wobei die Aminogruppe gegebenenfalls mit (C₁₋₆)Alkyl oder (C₁₋₆)Alkenyl substituiert ist, ausgewählt ist;
oder R⁶ und R⁸ zusammen eine Bindung bedeuten und R⁷ und R⁹ wie vorstehend definiert sind;
oder R⁶ und R⁸ zusammen -O- bedeuten und R⁷ und R⁹ beide Wasserstoff sind;
oder R⁶ und R⁷ oder R⁸ und R⁹ zusammen Oxo bedeuten;
und jedes R¹¹ unabhängig H, Trifluormethyl, (C₁₋₆)Alkyl, (C₁₋₆)Alkenyl, (C₁₋₆)Alkoxycarbonyl, (C₁₋₆)Alkylcarbonyl, Aminocarbonyl, wobei die Aminogruppe gegebenenfalls mit (C₁₋₆)Alkoxycarbonyl, (C₁₋₆)Alkylcarbonyl, (C₁₋₆)Alkenyloxycarbonyl, (C₂₋₆)Alkenylcarbonyl, (C₁₋₆)Alkyl oder (C₁₋₆)Alkenyl mono- oder disubstituiert ist, ist;
mit der Maßgabe, dass A und B nicht beide aus NR¹¹, O und S(O)ₓ ausgewählt sind und, wenn eines von A und B CO ist, das andere nicht CO, O oder S(O)ₓ ist;
wobei
"heterocyclisch" ein gegebenenfalls substituierter, aromatischer oder nicht aromatischer, einzelner oder kondensierter Ring ist, der in jedem Ring bis zu vier Heteroatome, ausgewählt aus Sauerstoff, Stickstoff und Schwefel, enthält und 4 bis 7 Ringatome aufweist, wobei die Ringe unsubstituiert oder mit bis zu drei Resten, ausgewählt aus Amino, Halogen, (C₁₋₆)Alkyl, (C₁₋₆)Alkoxy, Halogen(C₁₋₆)alkyl, Hydroxy, Carboxy, Carboxysalzen, (C₁₋₆)Alkoxycarbonyl, (C₁₋₆)Alkoxy carbonyl(C₁₋₆)alkyl, Aryl und Oxygruppen, substituiert sein können und wobei jede Aminogruppe, die Teil eines einzelnen oder kondensierten, nicht aromatischen, heterocyclischen, wie vorstehend definierten Rings bildet, gegebenenfalls substituiert ist mit (C₁₋₆)Alkyl, gegebenenfalls substituiert mit Hydroxy, (C₁₋₆)Alkoxy, Thiol, (C₁₋₆)Alkylthio, Halogen oder Trifluormethyl, Acyl- und (C₁₋₆)Alkylsulfonylresten;
"Aryl" gegebenenfalls substituiertes Phenyl oder Naphthyl ist, wobei gegebenenfalls substituiertes Phenyl oder Naphthyl gegebenenfalls mit bis zu fünf Resten, ausgewählt aus Halogen-, Mercapto-, (C₁₋₆)Alkyl-, Phenyl-, (C₁₋₆)Alkoxy-, Hydroxy(C₁₋₆)alkyl-, Mercapto(C₁₋₆)alkyl-, Halogen(C₁₋₆)alkyl-, Hydroxy-, Amino-, Nitro-, Carboxy-, (C₁₋₆)Alkylcarbonyloxy-, (C₁₋₆)Alkoxycarbonyl-, Formyl- und (C₁₋₆)Alkylcarbonylresten, substituiert ist;
"Acyl" (C₁₋₆)Alkoxycarbonyl, Formyl oder (C₁₋₆)Alkylcarbonyl ist.

2. Verbindung nach Anspruch 1, wobei Z¹ N ist und Z² bis Z⁵ jeweils CH sind oder Z⁵ N ist und Z¹ bis Z⁴ jeweils CH sind.

3. Verbindung nach Anspruch 1 oder 2, wobei R¹ Methoxy, Amino(C₃₋₅)alkyloxy, Guanidino(C₃₋₅)alkyloxy oder Fluor, am meisten bevorzugt Methoxy, ist.

4. Verbindung nach einem der vorstehenden Ansprüche, wobei R³ in der 3-Position ist und Aminocarbonyl(C₁₋₆)alkyl, Hydroxy(C₁₋₆)alkyl oder 1,2-Dihydroxy(C₂₋₆)alkyl ist, die gegebenenfalls an der (den) Hydroxygruppe(n) substituiert sind.

5. Verbindung nach einem der vorstehenden Ansprüche, wobei AB NHCO, NHCOCH₂ oder CH₂CH(OH)CH₂ ist.

6. Verbindung nach einem der vorstehenden Ansprüche, wobei R⁴ (C₅₋₁₀)Alkyl, unsubstituiertes Phenyl(C₂₋₃)alkyl oder unsubstituiertes Phenyl(C₃₋₄)alkenyl ist.

7. Verbindung nach Anspruch 1, ausgewählt aus:
[3R,4S]-1-Heptyl-4-[N-methyl-N-(6-methoxy-chinazolin-4-yl)-2-aminoethyl]-3-ethenylpiperidin;
[3R,4S]-1-Heptyl-4-[2-(6-methoxychinazolin-4-oxy)ethyl]-3-ethenylpiperidin;
1-Heptyl-4-(6-methoxy-1,5-naphthyridin-4-yl)aminocarbonylpiperidin;
[3R,4S]-1-Heptyl-3-ethenyl-4-N-(6-methoxy-1,5-naphthyridin-4-yl)-piperidinacetamid;
[3R,4S]-1-Heptyl-3-ethenyl-4-[2-(R,S)-hydroxy-3-(6-methoxy-1,5-naphthyridin-4-yl)propyl]piperidin;
[3R,4S]-1-Heptyl-4-N-(6-methoxy-1,5-naphthyridin-4-yl)-3,4-piperidindiacetamid;
[3R,4S]-1-Heptyl-4-N-(6-methoxy-1,5-naphthyridin-4-yl)-3-(1-(R,S)-2-dihydroxyethyl)-piperidinacetamid;
[3R,4S]-1-Heptyl-3-ethenyl-4-N-(6-methoxy-cinnolin-4-yl)-piperidinacetamid,
oder ein pharmazeutisch verträgliches Derivat jeder der vorstehenden Verbindungen.

8. Verfahren zur Herstellung von Verbindungen der Formel (I), oder eines pharmazeutisch verträglichen Derivats davon nach Anspruch 1, wobei das Verfahren umfasst:
(a) Umsetzen einer Verbindung der Formel (IV) mit einer Verbindung der Formel (V): 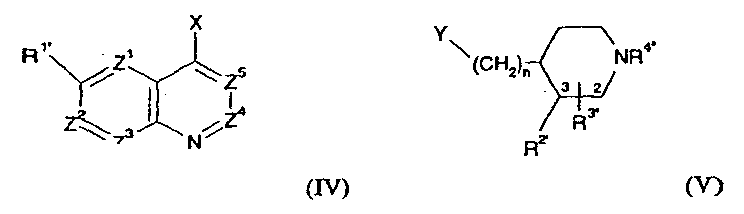 wobei Z¹, Z², Z³, Z⁴ und Z⁵, m, n, R¹, R², R³ und R⁴ wie in Formel (I) definiert sind und X und Y die folgenden Kombinationen sein können:
(i) X ist M und Y ist CH₂CO₂R^{x}
(ii) X ist CO₂R^{y} und Y ist CH₂CO₂R^{x}
(iii) eines von X und Y ist CH=SPh₂ und das andere ist CHO
(iv) X ist CH₃ und Y ist CHO
(v) X ist CH₃ und Y ist CO₂R^{x}
(vi) X ist CH₂CO₂R^{y} und Y ist CO₂R^{x}
(vii) X ist CH=PR^{z}₃ und Y ist CHO
(viii) X ist CHO und Y ist CH=PR^{z}₃
(ix) X ist Halogen und Y ist CH=CH₂
(x) eines von X und Y ist COW und das andere ist NHR^{11'}
(xi) eines von X und Y ist (CH₂)ₚ-V und das andere ist (CH₂)_{q}NHR^{11'}, (CH₂)_{q}OH, (CH₂)_{q}SH oder (CH₂)_{q}SCOR^{x}, wobei p+q=1 ist
(xii) eines von X und Y ist CHO und das andere ist NHR^{11'}
(xiii) eines von X und Y ist OH und das andere ist -CH=N₂
wobei V und W Abgangsgruppen sind, R^{x} und R^{y} (C₁₋₆)Alkyl sind und R^{z} Aryl oder (C₁₋₆)Alkyl ist;
oder
(b) Umsetzen einer Verbindung der Formel (IV) mit einer Verbindung der Formel (Vb): 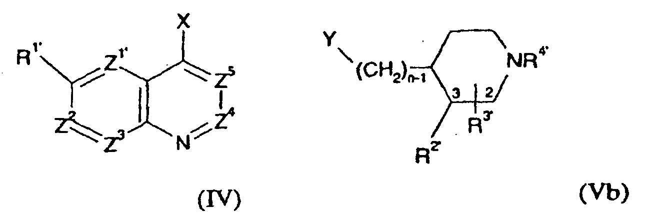 wobei Z¹, Z², Z³, Z⁴ und Z⁵, m, n, R¹, R², R³ und R⁴ wie in Formel (I) definiert sind, X CH₂NHR^{11'} ist und Y CHO oder COW ist oder X CH₂OH ist und Y -CH=N₂ ist;
wobei R^{11'}, R^{1'}, R^{2'}, R^{3'} und R^{4'} gleich R¹¹, R¹, R², R³ und R⁴ oder dazu konvertierbare Reste sind,
und danach gegebenenfalls oder wie notwendig Umwandeln von R^{11'}, R^{1'}, R^{2'}, R^{3'} und R^{4'} in R¹¹, R¹, R², R³ und R⁴, Umwandeln von A-B in ein anderes A-B, ineinander umwandeln von R¹¹, R¹, R², R³ und/oder R⁴ und Bilden eines pharmazeutisch verträglichen Derivats davon.

9. Arzneimittel, umfassend eine Verbindung der Formel (I) oder ein pharmazeutisch verträgliches Derivat davon nach Anspruch 1, und einen pharmazeutisch verträglichen Träger.

10. Verwendung einer Verbindung der Formel (I) oder eines pharmazeutisch verträglichen Derivats davon nach Anspruch 1 zur Herstellung eines Medikaments zur Verwendung bei der Behandlung von bakteriellen Infektionen in Säugern.

## Revendications

1. Composé de formule (I) ou un de ses dérivés pharmaceutiquement acceptables : 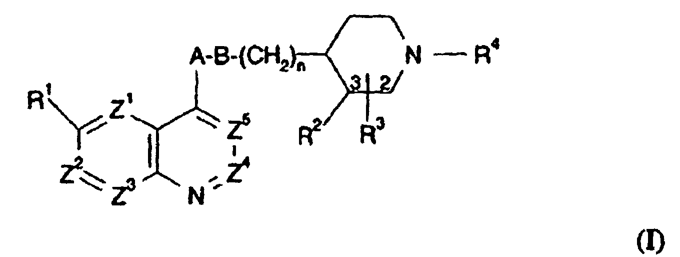 formule dans laquelle :
un des groupes Z¹, Z², Z³, Z⁴ et Z⁵ représente un atome de N et les groupes restants représentent des groupes CH ;
R¹ représente un atome d'hydrogène, un groupe hydroxy ; alkoxy en C₁ à C₆ facultativement substitué avec un substituant alkoxy en C₁ à C₆, amino, pipéridyle, guanidino ou amidino facultativement N-substitué avec un ou deux groupes alkyle en C₁ à C₆, acyle ou alkylsulfonyle en C₁ à C₆, NH₂CO, hydroxy, thiol, alkylthio en C₁ à C₆, hétérocyclylthio, hétérocyclyloxy, arylthio, aryloxy, acylthio, acyloxy ou alkylsulfonyloxy en C₁ à C₆ ; alkyle en C₁ à C₆ à substituant alkoxy en C₁ à C₆ ; halogéno ; alkyle en C₁ à C₆ ; alkylthio en C₁ à C₆ ; nitro ; trifluorométhyle ; azido, acyle ; acyloxy ; acylthio ; alkylsulfonyle en C₁ à C₆ ; alkylsulfoxyde en C₁ à C₆ ; arylsulfonyle ; arylsulfoxyde, ou un groupe amino, pipéridyle, guanidino ou amidino facultativement N-substitué avec un ou deux groupes alkyle en C₁ à C₆, acyle ou alkylsulfonyle en C₁ à C₆ ;
soit R² représente un atome d'hydrogène ; et
R³ est en position 2 ou 3 et représente un atome d'hydrogène ou un groupe alkyle en C₁ à C₆ ou alcényle en C₂ à C₆ facultativement substitué avec un à trois groupes choisis entre :
thiol ; halogéno ; alkylthio en C₁ à C₆ ; trifluorométhyle ; azido ; (alkoxy en C₁ à C₆)carbonyle ; (alkyle en C₁ à C₆)carbonyle ; (alcényle en C₂ à C₆)oxycarbonyle ; (alcényle en C₂ à C₆)carbonyle ; hydroxy facultativement substitué avec un substituant alkyle en C₁ à C₆, alcényle en C₂ à C₆, (alkoxy en C₁ à C₆)carbonyle, (alkyle en C₁ à C₆)carbonyle, (alcényle en C₂ à C₆)oxycarbonyle, (alcényle en C₂ à C₆)carbonyle ou aminocarbonyle dans lequel le groupe amino est facultativement substitué avec un substituant alkyle en C₁ à C₆, alcényle en C₂ à C₆, (alkyle en C₁ à C₆)carbonyle ou (alcényle en C₂ à C₆)carbonyle ; amino facultativement mono- ou disubstitué avec un substituant (alkoxy en C₁ à C₆)carbonyle, (alkyle en C₁ à C₆)carbonyle, (alcényle en C₂ à C₆)oxycarbonyle, (alcényle en C₂ à C₆)carbonyle ; alkyle en C₁ à C₆, alcényle en C₂ à C₆, alkylsulfonyle en C₁ à C₆, alcénylsulfonyle en C₂ à C₆ ou aminocarbonyle dans lequel le groupe amino est facultativement substitué avec un substituant alkyle en C₁ à C₆ ou alcényle en C₂ à C₆ ; aminocarbonyle dans lequel le groupe amino est facultativement mono- ou disubstitué avec un substituant alkyle en C₁ à C₆, alcényle en C₂ à C₆, (alkoxy en C₁ à C₆)carbonyle, (alkyle en C₁ à C₆)carbonyle, (alcényle en C₂ à C₆)oxycarbonyle ou (alcényle en C₂ à C₆)carbonyle ; oxo ; alkylsulfonyle en C₁ à C₆ ; alcénylsulfonyle en C₂ à C₆ ; ou aminosulfonyle en C₁ à C₆ dans lequel le groupe amino est facultativement substitué avec un substituant alkyle en C₁ à C₆ ou alcényle en C₂ à C₆ ; soit
R³ est en position 3 et R² et R³, conjointement, représentent un résidu divalent de =CR^{5¹}R^{6¹} dans lequel R^{5¹} et R^{6¹} sont choisis indépendamment entre H, des groupes alkyle en C₁ à C₆, alcényle en C₂ à C₆, aryl (alkyle en C₁ à C₆) et aryl(alcényle en C₂ à C₆), n'importe quel groupement alkyle ou alcényle étant facultativement substitué avec un à trois groupes choisis parmi ceux énumérés ci-dessus pour les substituants sur R³ ;
R⁴ représente un groupe -CH₂R⁵ dans lequel R⁵ est choisi entre :
alkyle en C₃ à C₁₂ ; hydroxyalkyle en C₃ à C₁₂ ; (alkoxy en C₁ à C₁₂) (alkyle en C₃ à C₁₂) ; (alcanoyle en C₁ à C₁₂)oxy(alkyle en C₃ à C₁₂) ; (cycloalkyle en C₃ à C₆) (alkyle en C₃ à C₁₂) ; hydroxy-, (alkoxy en C₁ à C₁₂) - ou (alcanoyle en C₁ à C₁₂)oxy-(cycloalkyle en C₃ à C₆)(alkyle en C₃ à C₁₂) ; cyano(alkyle en C₃ à C₁₂) ; alcényle en C₂ à C₁₂ ; alcynyle en C₂ à C₁₂ ; tétrahydrofuryle ; mono- ou di(alkyle en C₁ à C₁₂)amino(alkyle en C₃ à C₁₂) ; acylamino(alkyle en C₃ à C₁₂) ; (alkyle en C₁ à C₁₂) - ou acylaminocarbonyl(alkyle en C₃ à C₁₂) ; mono- ou di (alkyle en C₁ à C₁₂)amino(hydroxy) (alkyle en C₃ à C₁₂) ; phényl (alkyle en C₁ ou C₂) facultativement substitué, phénoxy(alkyle en C₁ ou C₂) ou phényl (hydroxy) (alkyle en C₁ ou C₂) ; diphényl (alkyle en C₁ ou C₂) facultativement substitué ; phényl(alcényle en C₂ ou C₃) facultativement substitué ; benzoyle ou benzoylméthyle facultativement substitué ; hétéroaryl (alkyle en C₁ ou C₂) facultativement substitué ; et hétéroaroyle ou hétéroaroylméthyle facultativement substitué ;
n est égal à 0, 1 ou 2 ;
A représente un groupe NR¹¹, O, S(O)ₓ ou CR⁶R⁷ et B représente un groupe NR¹¹, O, S(O)ₓ ou CR⁸R⁹ dans lequel x est égal à 0, 1 ou 2, et groupes dans lesquels :
chacun de R⁶ et R⁷, R⁸ et R⁹, est choisi indépendamment entre H ; thiol ; alkylthio en C₁ à C₆ ; halogéno ; trifluorométhyle ; azido ; alkyle en C₁ à C₆ ; alcényle en C₂ à C₆ ; (alkoxy en C₁ à C₆)carbonyle ; (alkyle en C₁ à C₆)carbonyle ; (alcényle en C₂ à C₆)oxycarbonyle ; (alcényle en C₂ à C₆)carbonyle ; hydroxy, amino ou aminocarbonyle facultativement substitué comme pour les substituants correspondants dans R³ ; alkylsulfonyle en C₁ à C₆ ; alcénylsulfonyle en C₂ à C₆ ou aminosulfonyle en C₁ à C₆ dans lequel le groupe amino est facultativement substitué avec un substituant alkyle en C₁ à C₆ ou alcényle en C₁ à C₆ ;
ou R⁶ et R⁸, conjointement, représentent une liaison et R⁷ et R⁹ répondent aux définitions précitées ;
ou R⁶ et R⁸, conjointement, représentent un groupe -O- et R⁷ et R⁹ représentent l'un et l'autre un atome d'hydrogène ;
ou bien R⁶ et R⁷ ou R⁸ et R⁹, conjointement, représentent un groupe oxo ;
et chaque groupe R¹¹ représente indépendamment H, un groupe trifluorométhyle, alkyle en C₁ à C₆, alcényle en C₁ à C₆, (alkoxy en C₁ à C₆)carbonyle, (alkyle en C₁ à C₆)carbonyle, aminocarbonyle dans lequel le groupe amino est facultativement mono- ou disubstitué avec un substituant (alkoxy en C₁ à C₆)carbonyle, (alkyle en C₁ à C₆)carbonyle, (alcényle en C₁ à C₆)oxycarbonyle, (alcényle en C₂ à C₆)carbonyle, alkyle en C₁ à C₆ ou alcényle en C₁ à C₆;
sous réserve que A et B ne puissent être tous deux choisis entre des groupes NR¹¹, O et S(O)ₓ et, lorsqu'un de A et B représente un groupe CO, l'autre ne représente pas un groupe CO, O ou S(O)ₓ ;
où le terme "hétérocyclique" désigne un noyau aromatique ou non aromatique, unique ou condensé, facultativement substitué, contenant dans chaque noyau jusqu'à 4 hétéroatomes choisis entre des atomes d'oxygène, d'azote et de soufre, et ayant 4 à 7 atomes de noyau, noyaux qui peuvent être non substitués ou substitués avec jusqu'à trois groupes choisis entre des groupes amino, halogéno, alkyle en C₁ à C₆, alkoxy en C₁ à C₆, halogénalkyle en C₁ à C₆, hydroxy, carboxy, sels carboxyliques, (alkoxy en C₁ à C₆)carbonyle, (alkoxy en C₁ à C₆)carbonyl (alkyle en C₁ à C₆), aryle et oxo, et n'importe quel groupe amino faisant partie d'un noyau hétérocyclique non aromatique unique ou condensé, de la manière définie ci-dessus, étant facultativement substitué avec un groupe alkyle en C₁ à C₆ facultativement substitué avec des groupes hydroxy, alkoxy en C₁ à C₆, thiol, alkylthio en C₁ à C₆, halogéno ou trifluorométhyle, acyle et alkylsulfonyle en C₁ à C₆ ;
le terme "aryle" désigne un groupe phényle ou naphtyle facultativement substitué ;
le groupe phényle ou naphtyle facultativement substitué est facultativement substitué avec jusqu'à cinq groupes choisis entre des groupes halogéno, mercapto, alkyle en C₁ à C₆, phényle, alkoxy en C₁ à C₆, hydroxyalkyle en C₁ à C₆, mercaptoalkyle en C₁ à C₆, halogénalkyle en C₁ à C₆, hydroxy, amino, nitro, carboxy, (alkyle en C₁ à C₆)carbonyloxy, (alkoxy en C₁ à C₆)carbonyle, formyle et (alkyle en C₁ à C₆)carbonyle ;
le terme "acyle" désigne un groupe (alkoxy en C₁ à C₆)carbonyle, formyle ou (alkyle en C₁ à C₆)carbonyle.

2. Composé suivant la revendication 1, dans lequel Z¹ représente un atome de N et Z²-Z⁵ représentent chacun un groupe CH ou Z⁵ représente un atome de N et Z¹-Z⁴ représentent chacun un groupe CH.

3. Composé suivant la revendication 1 ou 2, dans lequel R¹ représente un groupe méthoxy, amino(alkyle en C₃ à C₅)oxy, guanidino(alkyle en C₃ à C₅)oxy ou fluoro, de préférence méthoxy.

4. Composé suivant l'une quelconque des revendications précédentes, dans lequel R³ est en position 3 et représente un groupe aminocarbonyl(alkyle en C₁ à C₆), hydroxyalkyle en C₁ à C₆ ou 1,2-dihydroxy(alkyle en C₂ à C₆) facultativement substitué sur le ou les groupes hydroxy.

5. Composé suivant l'une quelconque des revendications précédentes, dans lequel AB représente un groupe NHCO, NHCOCH₂ ou CH₂CH(OH)CH₂.

6. Composé suivant l'une quelconque des revendications précédentes, dans lequel R⁴ représente un groupe alkyle en C₅ à C₁₀, phényl (alkyle en C₂ ou C₃) non substitué ou phényl(alcényle en C₃ ou C₄) non substitué.

7. Composé suivant la revendication 1, choisi entre :
[3R,4S]-1-heptyl-4-[N-méthyl-N-(6-méthoxyquinazoline-4-yl)-2-aminoéthyl]-3-éthénylpipéridine ;
[3R,4S]-1-heptyl-4-[2-(6-méthoxyquinazoline-4-oxy)-éthyl]-3-éthénylpipéridine ;
1-heptyl-4-(6-méthoxy-1,5-naphtyridine-4-yl)aminocarbonylpipéridine ;
[3R,4S]-1-heptyl-3-éthényl-4-N-(6-méthoxy-1,5-naphtyridine-4-yl)pipéridine-acétamide ;
[3R,4S]-1-heptyl-3-éthényl-4-[2-(R,S)-hydroxy-3-(6-méthoxy-1,5-naphtyridine-4-yl)propyl]pipéridine ;
[3R,4S]-1-heptyl-4-N-(6-méthoxy-1,5-naphtyridine-4-yl)-3,4-pipéridine-acétamide ;
[3R,4S]-1-heptyl-4-N-(6-méthoxy-1,5-naphtyridine-4-yl)-3-(1-(R,S)-2-dihydroxyéthyl)pipéridine-acétamide ;
[3R,4S]-1-heptyl-3-éthényl-4-N-(6-méthoxycinnoline-4-yl)pipéridine-acétamide [ou un dérivé pharmaceutiquement acceptable de n'importe lequel des composés précités].

8. Procédé pour la préparation de composés de formule (I), ou d'un de leurs dérivés pharmaceutiquement acceptables suivant la revendication 1, procédé qui comprend :
(a) la réaction d'un composé de formule (IV) avec un composé de formule (V) : 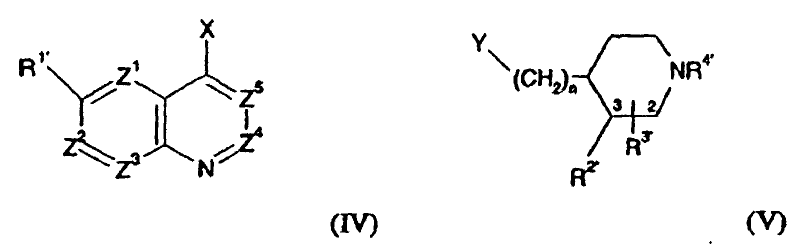 formules dans lesquelles Z¹, Z², Z³, Z⁴ et Z⁵, m, n, R¹, R², R³ et R⁴ répondent aux définitions indiquées pour la formule (I), et X et Y peuvent représenter les associations suivantes :
(i) X représente M et Y représente un groupe CH₂CO₂R^{x}
(ii) X représente un groupe CO₂R^{y} et Y représente un groupe CH₂CO₂R^{x}
(iii) un de X et Y représente un groupe CH=SPh₂ et l'autre représente un groupe CHO
(iv) X représente un groupe CH₃ et Y représente un groupe CHO
(v) X représente un groupe CH₃ et Y représente un groupe CO₂R^{x}
(vi) X représente un groupe CH₂CO₂R^{y} et Y représente un groupe CO₂R^{x}
(vii) X représente un groupe CH=PR^{z}₃ et Y représente un groupe CHO
(viii) X représente un groupe CHO et Y représente un groupe CH=PR^{z}₃
(ix) X représente un atome d'halogène et Y représente un groupe CH=CH₂
(x) l'un de X et Y représente l'un groupe COW et l'autre représente un groupe NHR^{11'}
(xi) l'un de X et Y représente l'un groupe (CH₂)ₚ-V et l'autre représente un groupe (CH₂)_{q}NHR^{11'}, (CH₂)_{q}OH, (CH₂)_{q}SH ou (CH₂)_{q}SCOR^{x} dans lequel p + q = 1
(xii) l'un de X et Y représente l'un groupe CHO et l'autre représente un groupe NHR^{11'}
(xiii) l'un de X et Y représente l'un groupe OH et l'autre représente un groupe -CH=N₂
dans lesquelles V et W représentent des groupes partants, R^{x} et R^{y} représentent des groupes alkyle en C₁ à C₆ et R^{z} représente un groupe aryle ou alkyle en C₁ à C₆ ;
ou
(b) la réaction d'un composé de formule (IV) avec un composé de formule (Vb) : 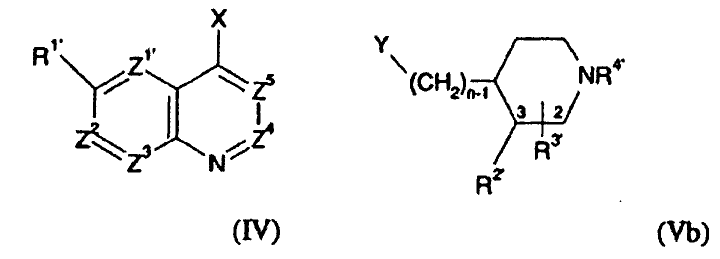 formules dans lesquelles Z¹, Z², Z³, Z⁴ et Z⁵, m, n, R¹, R², R³ et R⁴ répondent aux définitions indiquées pour la formule (I), X représente un groupe CH₂NHR^{11'} et Y représente un groupe CHO ou COW, ou X représente un groupe CH₂OH et Y représente un groupe -CH=N₂ ;
groupes dans lesquels R^{11'}, R^{1'}, R^{2'}, R^{3'} et R^{4'} représentent des groupes R¹¹, R¹, R², R³ et R⁴ ou des groupes pouvant être convertis en ceux-ci, et ensuite, facultativement ou de la manière nécessaire, la conversion de R^{11'}, R^{1'}, R^{2'}, R^{3'} et R^{4'} en des groupes R¹¹, R¹, R², R³ et R⁴, la conversion du groupe A-B en un autre groupe A-B, l'interconversion de R¹¹, R¹, R², R³ et/ou R⁴ et la formation d'un dérivé pharmaceutiquement acceptable du composé obtenu.

9. Composition pharmaceutique comprenant un composé de formule (I) ou un de ses dérivés pharmaceutiquement acceptables suivant la revendication 1, et un support pharmaceutiquement acceptable.

10. Utilisation d'un composé de formule (I) ou d'un de ses dérivés pharmaceutiquement acceptables suivant la revendication 1 dans la production d'un médicament destiné à être utilisé dans le traitement d'infections bactériennes chez des mammifères.
